# EUROPEAN PATENT SPECIFICATION

(11) **EP 3 997 214 B1**
(45) Date of publication and mention of the grant of the patent: **02.08.2023**
(21) Application number: 20736736.8
(22) Date of filing: 10.07.2020
(51) Int. Cl.: C12N 7/00, C07K 14/015, A61K 48/00

(54) **CHEMICALLY-MODIFIED ADENO-ASSOCIATED VIRUS**
CHEMISCH MODIFIZIERTER ADENO-ASSOZIIERTER VIRUS
VIRUS ADÉNO-ASSOCIÉ CHIMIQUEMENT MODIFIÉ

(30) Priority: 11.07.2019 EP 19185879
(43) Date of publication of application: 18.05.2022
(73) Proprietor: Centre National de la Recherche Scientifique, 75016 Paris (FR); Nantes Université, 44000 Nantes (FR); INSERM (Institut National de la Santé et de la Recherche Médicale), 75013 Paris (FR); Centre Hospitalier Universitaire De Nantes, 44000 Nantes (FR)
(72) Inventor: DENIAUD, David, 44800 Saint Herblain (FR); MEVEL, Mathieu, 44300 Nantes (FR); AYUSO, Eduard, 44100 Nantes (FR); LERAY, Aurélien, 44000 Nantes (FR)
(74) Representative: Cabinet Becker et Associés
(86) International application number: PCT/EP2020/069554
(87) International publication number: WO 2021/005210

(56) References cited:
- EP-A1- 3 461 836
- WO-A1-2017/212019
- HOOKER JACOB M ET AL: "Interior surface modification of bacteriophage MS2", JOURNAL OF THE AMERICAN CHEMICAL SOCIETY, AMERICAN CHEMICAL SOCIETY, US, vol. 126, no. 12, 31 March 2004 (2004-03-31), pages 3718-3719, XP002461219, ISSN: 0002-7863, DOI: 10.1021/JA031790Q
- MICHAEL A. BRUCKMAN ET AL: "Surface Modification of Tobacco Mosaic Virus with "Click" Chemistry", CHEMBIOCHEM, vol. 9, no. 4, 3 March 2008 (2008-03-03), pages 519-523, XP055627885, ISSN: 1439-4227, DOI: 10.1002/cbic.200700559
- KAI LI ET AL: "Chemical Modification of M13 Bacteriophage and Its Application in Cancer Cell Imaging", BIOCONJUGATE CHEMISTRY, vol. 21, no. 7, 21 July 2010 (2010-07-21), pages 1369-1377, XP055056043, ISSN: 1043-1802, DOI: 10.1021/bc900405q
- XIA ZHAO ET AL: "Enhancing Antibody Response against Small Molecular Hapten with Tobacco Mosaic Virus as a Polyvalent Carrier", CHEMBIOCHEM, vol. 16, no. 9, 15 June 2015 (2015-06-15), pages 1279-1283, XP055628052, ISSN: 1439-4227, DOI: 10.1002/cbic.201500028
- HITOSHI BAN ET AL: "Facile and Stabile Linkages through Tyrosine: Bioconjugation Strategies with the Tyrosine-Click Reaction", BIOCONJUGATE CHEMISTRY, vol. 24, no. 4, 17 April 2013 (2013-04-17) , pages 520-532, XP055405924, US ISSN: 1043-1802, DOI: 10.1021/bc300665t
- JULIA GAVRILYUK ET AL: "Formylbenzene Diazonium Hexafluorophosphate Reagent for Tyrosine-Selective Modification of Proteins and the Introduction of a Bioorthogonal Aldehyde", BIOCONJUGATE CHEMISTRY, vol. 23, no. 12, 29 November 2012 (2012-11-29), pages 2321-2328, XP055627630, US ISSN: 1043-1802, DOI: 10.1021/bc300410p

## Description

### Field of the invention

The invention relates to chemically modified adeno-associated (AAV) virus and their use in gene therapy.

### Background of the invention

Gene therapy was originally developed to correct defective genes that underlie genetic diseases. Nowadays, gene therapy is more and more used in the treatment of a broad range of acquired diseases such as cancers.

Gene therapy is based on the therapeutic delivery of nucleic acid into a patient's cell nucleus. The nucleic acids may then be inserted into the genome of the targeted cell or may remain episomal. Delivery of a therapeutic nucleic acid to a subject's target cells can be carried-out by various methods, including the use of synthetic and viral vectors. Among the many viral vectors available (e.g, retrovirus, lentivirus, adenovirus, and the like), recombinant adeno-associated virus (AAV) is gaining popularity as a versatile vector for gene therapy, particularly for in vivo applications. The main advantages of recombinant AAV (rAAV) reside in their broad tropism, their high transduction efficacy, their persistent episomal expression and their high safety profile, in particular because wild-type AAV is not associated with any human diseases. Human clinical trials with rAAV have demonstrated durable expression at therapeutic levels when targeting tissues such as retina, liver or motor neurons. Several clinical trials using rAAV as gene vector are ongoing for a wide type of disorders. The FDA and the EMA have recently authorized Voretigene neparvovec (Luxturna^{®}), which is an adeno-associated viral vector serotype 2 (AAV2) capsid comprising a cDNA encoding for the human retinal pigment epithelium 65kDa protein (hRPE65), for the treatment of vision loss due to inherited retinal dystrophy caused by confirmed biallelic RPE65 mutations. As a further example, Zolgensma^{®} (onasemnogene abeparvovec-xioi) has just been approved by the FDA for the treatment of pediatric patients less than 2 years of age with spinal muscular atrophy (SMA). Zolgensma^{®} is a AAV9 vector able to deliver a functional, non-mutated copy of the defective gene in SMA, namely the *SMN1* gene, in motoneurons.

In spite of these success, certain clinical trials have shown some limitations of these vectors, in the treatment of certain diseases. Their first limitation lies on their immunogenicity. Because of their non-integrative nature, systemic gene therapy with AAV vectors, especially in paediatric patients, might be limited by tissue proliferation inducing a dilution of the vector over time. However, the re-administration of the vectors might be precluded by persistent neutralizing antibodies (Nabs) triggered following the first administration of the viral vector. Moreover, it was further shown that preexisting humoral immunity to certain AAV serotypes, especially AAV of serotype 2, are highly prevalent in humans. Anti-AAV neutralizing antibodies (NAbs) can completely prevent transduction in a target tissue, resulting in lack of efficacy, particularly when the vector is administered directly into the bloodstream. As a result, subjects seropositive to AAV-Nabs are generally excluded from gene therapy trials.

A further limitation of AAV lies on their broad tropism, which may result in transgene expression in other tissues other than those where transgene expression is desired.

AAV as gene vector may also suffer from a reduced therapeutic index. Sometimes, the administration of high dose of AAV is needed to achieve effective transduction. For instance, although AAV2 vectors can efficiently target the liver, the transgene expression can be restricted to a very small of the transfected hepatocytes due to intracellular proteasome-mediated degradation of the vectors, whereby high dose or AAV-2 may be required to achieve the sought therapeutic effect. Such high doses pose a challenge not only for vector production but also increases the risk of immune response, among which the induction of Nabs.

Several strategies have been proposed to overcome the drawbacks of AAV, especially those of the AAV of serotype 2 (AAV2) in gene therapy. Certain of them are based on the modification of the capsid proteins of the vectors.

In that matter, it was shown that mutations in surface-exposed tyrosine residues on AAV2 enable to circumvent phosphorylation and subsequent ubiquitination thereby avoiding proteasome-mediated degradation. Indeed, the lack of capsid ubiquitination is believed to improve the intracellular trafficking of the viral vectors into the nucleus, resulting in an increase in transduction efficiency in hepatocytes both *in vitro* and *in vivo* (Zhong et al., PNAS, 2008,105, 7827-7832; Markusic et al. Molecular Therapy, 2010, 18, 2048-2056). Similar results were observed for AAV8 in gene transfer efficiency in retinal cells (Petrs-Silva, 2009, Molecular Therapy, 17:463-471).

Chemical modifications of the viral capsids were also proposed in order to introduce a ligand on the capsid or mask certain exposed amino acids so as to modify the antigenicity, the tropism or the transduction efficacity of AAV. As a first strategy, several studies have explored the genetic incorporation of unnatural amino acids with modified side chains comprising a reactive group such as alkyne, azido or aminophenyl groups enabling the subsequent selective coupling of a ligands by orthogonal reactions. For instance, in WO2015/062516, a non-natural amino acid, such as an amino acid comprising an azido, is inserted into the capsid by genetic modification prior to a coupling step with a ligand by click reaction so as to change its tropism for the target cell.

Another strategy resides in the direct chemical modification of the viral capsid without any preliminary site-directed mutagenesis of the capsid proteins.

In that matter, Lee et al. (Biotechnol Bioeng 2005, 92:24-34) examine the potential of conjugating the AAV surface with activated polyethylene glycol chains to protect the vector from neutralizing antibodies. Horowitz et al. (Bioconj Chem, 2011, 22(4):529-532) describe a chemical approach for selectively masking arginine residues on viral capsid based on glycation with methylglyoxal. The resulting chemically-modified AAV retained ability to infect neurons in mouse brain and were redirected from liver to skeletal and cardiac muscle following systemic infection in mice. Furthermore, these glycated AAV displayed altered antigenicity which might enable to evade antibody neutralization. At last, International patent application WO2017/212019 proposes a method for chemically modifying the AAV capsid by covalently coupling a ligand bearing an isothiocyanate group which reacts with an amino group present in an amino acid residue such as lysine or arginine.

However, there is still a need for new methods enabling to modulate the properties of AAV when used as gene delivery vectors in gene therapy.

### Summary of the invention

The invention relates to an adeno-associated Virus (AAV) having at least one chemically-modified tyrosine residue in its capsid, wherein said chemically-modified tyrosine residue is of formula (I):

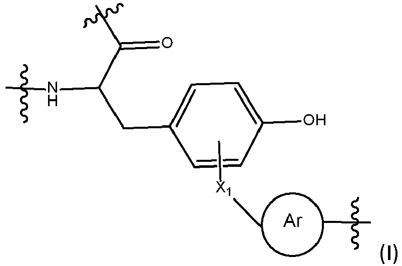

Wherein:
- X₁ is selected from the group consisting of: 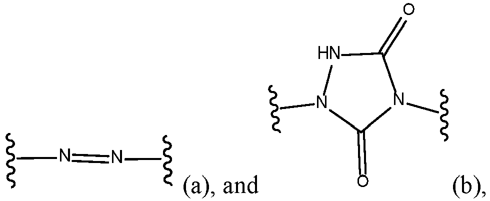 and
- Ar is an aryl or a heteroaryl moiety optionally substituted.

X₁ is preferably at position ortho of the phenol group.

In some embodiments, the at least one chemically-modified tyrosine residue is of formula (Ia): 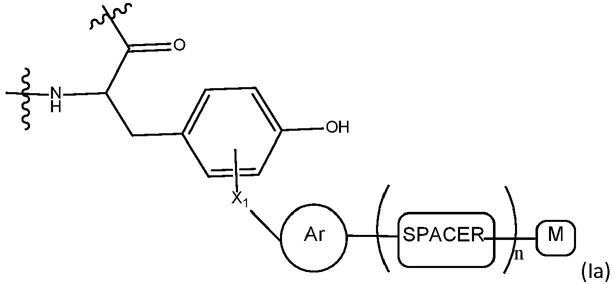 Wherein
- X₁, and Ar are as defined above,
- Spacer is a group for linking the "Ar" group to the functional moiety "M" which preferably comprises up to 1000 carbon atoms and which is preferably in the form of a chemical chain which optionally comprises heteroatoms and/or cyclic moieties,
- n is 0 or 1, and
- M is a functional moiety comprising a group selected from a steric shielding agent, a labelling agent, cell-type specific ligand, a drug moiety and combinations thereof.

In some particular embodiments, X₁ is of formula (a) and/or "Ar" is selected from substituted or unsubstituted phenyl, pyridyl, naphthyl, and anthracenyl.

In some other embodiments, the at least one chemically-modified tyrosine in the capsid of the AAV is of formula (1c): 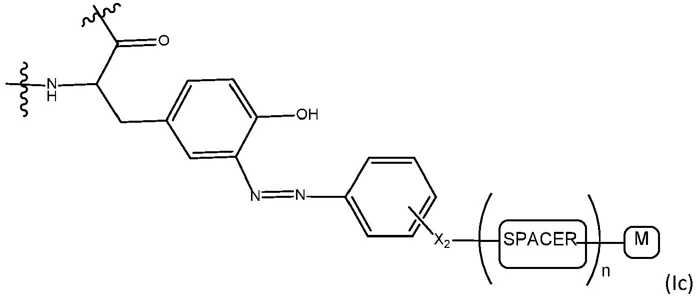 wherein:
- X₂ is -C(=O)-NH, -C(=O)-O, -C(=O)-O-C(=O)-, O-(C=O)-, NH-C(=O)-, NH-C(=O)-NH, - O-C=O-O-, O, NH, -NH(C=S)-, or -(C=S)-NH-, preferably -(C=O)-NH- or -(C=O)-O-
- X₂ is at position para, meta or ortho, preferably at position para of the phenylene group,
- Spacer, n and M are as defined above.

"Spacer", when present, may be selected from the group consisting of saturated or unsaturated, linear or branched C₂-C₄₀ hydrocarbon chains, optionally substituted, polyethylene glycol, polypropylene glycol, pHPMA, PLGA, polymers of alkyl diamines and combinations thereof, and/or "M" may comprise, or consist of, cell-type targeting ligand, preferably selected from a mono- or a polysaccharide, a hormone, including a steroid hormone, a peptide such as RGD peptide, a muscle targeting peptide (MTP) or Angiopep-2, a protein or a fragment thereof, a membrane receptor or a fragment thereof, an aptamer, an antibody including heavy-chain antibody, and fragments thereof such as Fab, Fab', and VHH, a ScFv, a spiegelmer, a peptide aptamer, vitamins and drugs such as CB1 and/or CB2 ligands.

In some other embodiments, "Spacer" (when present) is selected from the group consisting of linear or branched C₂-C₂₀ alkyl chains, polyethylene glycol, polypropylene glycol, pHPMA, PLGA, polymer of alkyl diamine and combinations thereof, said polymers having from 2 to 20 monomers and/or "M" comprises, or consists of, a cell-type specific ligand derived from a protein selected from transferrin, Epidermal Growth Factor (EGF), and basic Fibroblast Growth Factor βFGF, a mono- or a polysaccharide comprising one or several galactose, mannose, N-acetylgalactosamine residues, bridge GalNac, or mannose-6-phosphate, a MTP selected from SEQ ID NO: 1 to SEQ ID NO:7, and vitamins such as folic acid.

For instance, M is a cell-type specific ligand for specifically targeting hepatocytes and comprises at least one moiety of formula (III): 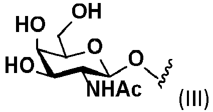 and/or "Spacer" is a polyethylene glycol chain comprising from 2 to 10 monomers.

As a further example, the AAV comprises at least one chemically modified tyrosine in its capsid which is of formula (II):

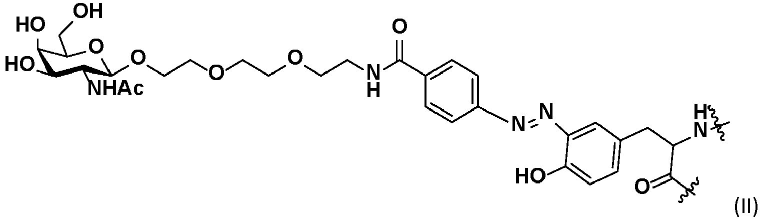

In some other embodiments, the AAV of the invention further has at least one additional chemically modified amino acid residue in the capsid, which is different from a tyrosine residue, said amino acid residue preferably bearing an amino group chemically modified with a group of formula (V): 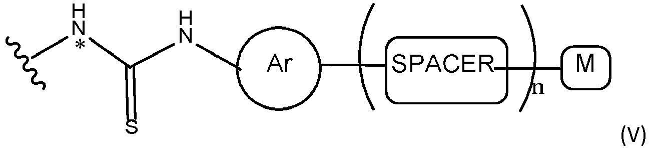 Wherein:
- N* being the nitrogen of the amino group of an amino acid residue, e.g of a lysine residue or arginine residue,
- Ar, Spacer, n and M has the same definition as Ar, Spacer, n and M as defined in formula (II) above.

The AAV of the invention may be of any type. For instance, the AAV may be a recombinant AAV, preferably selected from AAV having a wildtype capsid, naturally-occurring serotype AAV, variant AAV, pseudotype AAV, AAV with hybrid or mutated capsids, and self-complementary AAV.

The invention also relates to a method for chemically-modifying the capsid of an AAV, more precisely for chemically modifying at least one tyrosine residue in the capsid of an AAV, which comprises incubating said AAV with a chemical reagent bearing a reactive group selected from an aryl diazonium salt, and a 4-phenyl-1,2,4-triazole-3,5-dione (PTAD) moiety in conditions conducive for reacting said reactive group with a tyrosine residue present in the capsid of the AAV so as to form a covalent bound.

In some embodiments, the method of the invention comprises incubating the AAV with a chemical reagent of formula (VId) 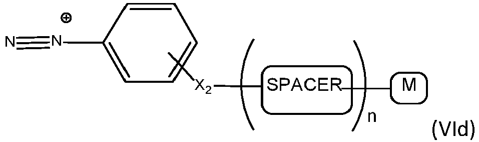 so as to obtain at least one chemically-modified tyrosine residue in the capsid of formula (Ic):

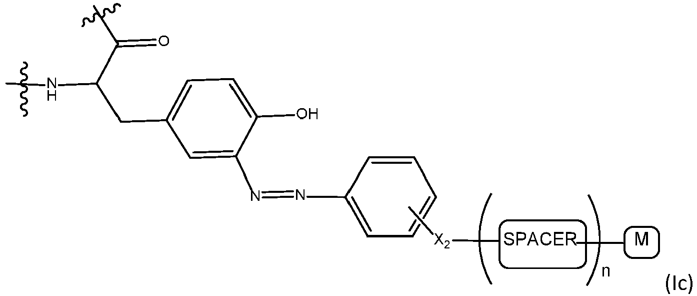

Wherein:
- X₂ is -C(=O)-NH, -C(=O)-O, -C(=O)-O-C(=O)-, O-(C=O)-, NH-C(=O)-, NH-C(=O)-NH, - O-C=O-O-, O, NH, -C=S-NH, or NH-C=S- preferably -(C=O)-NH- or -(C=O)-O-
- X₂ is at position para, meta or ortho, preferably at position para of the phenyl group,
- Spacer is a hydrocarbon group comprising from 2 to 400 carbon atoms,
- n is 0 or 1, and
- M is a chemical moiety comprising a steric shielding agent, a labelling agent, cell-type specific ligand or a drug moiety.

The invention also relates to an AAV obtainable or obtained by the method of the invention. A further object of the invention is a pharmaceutical composition comprising an AAV as defined above and at least one pharmaceutically acceptable excipient. The Invention further relates to said AAV or said pharmaceutical composition for use as a diagnostic agent or as a drug *in vivo,* preferably in gene therapy. The Invention also relates to the use of said AAV, or said pharmaceutical composition as a diagnostic agent and as a gene vector *ex vivo* or *in vitro.*

### Figures

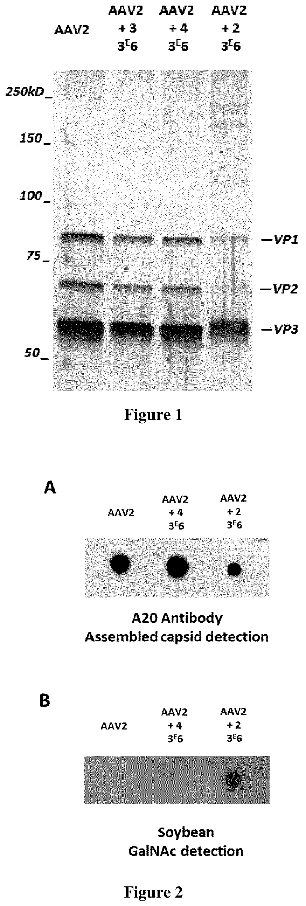

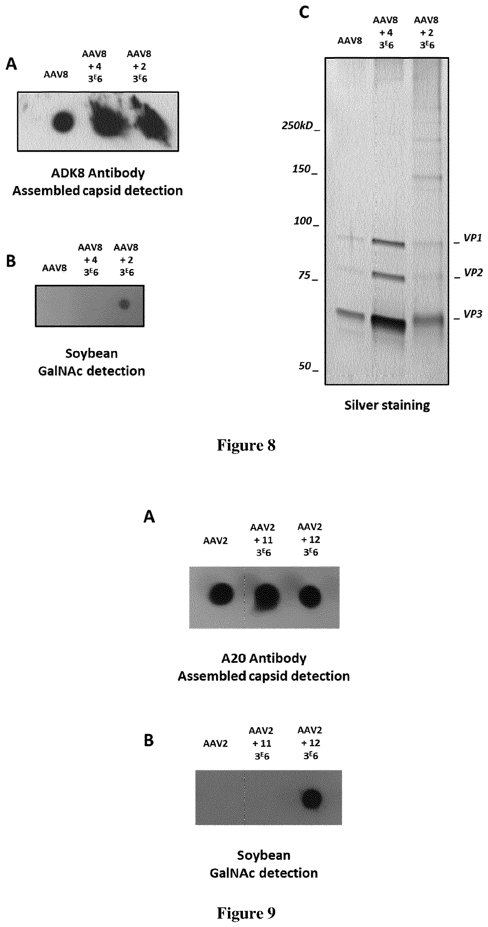

Figure 1, Figure 4C, Figure 6C, Figure 7C and Figure 8C: AAV capsid protein integrity measured by SDS-PAGE and silver staining. 10¹² vg of AAV2-Luc-GFP, AAV2-GFP and AAV8-GFP vectors was added to a solution of **2, 3, 4, 6, 7 or 8** (3E5 or 3E6 eq) in TBS buffer (pH 9.3) and incubated for 4 h at RT. 10¹⁰ vg of each condition was analyzed by SDS-PAGE and silver staining. VP1, VP2 and VP3 are the three proteins constituting the AAV capsid. Capsid protein molecular weight is indicated at the right of the images according to a protein ladder. AAV2 +2: AAV2 incubated with compound **2** (invention), AAV2 +3: AAV2 incubated with compound **3** (comparative), AAV2 +4: AAV2 incubated with compound **4** (comparative), AAV2 +6: AAV2 incubated with compound **6** (invention), AAV2 +7: AAV2 incubated with compound **7** (invention), AAV2 +8: AAV2 incubated with compound **8** (comparative), AAV8 +4: AAV8 incubated with compound **4** (comparative), AAV8 +2: AAV2 incubated with compound **2** (invention).
Figure 2, Figure 5, Figure 6, Figure 7, Figure 8 and Figure 9: dot blot analysis. 10¹² vg of AAV2-Luc-GFP, AAV2-GFP or AAV8-GFP vectors was added to a solution of compounds **2, 3, 4, 6, 7, 8, 9,10, 11** and **12** (3E5 or 3E6 eq) in TBS buffer (pH 9.3) and incubated for 4 h at RT. (**A-B**) 10⁹ vg of each condition was analyzed by dot blot using the A20 or ADK8 antibody that recognizes the assembled capsid (**A**), or using a FITC-soybean lectin or FITC-concanavalin A lectin that recognizes the GalNAc and mannose sugar (**B**).

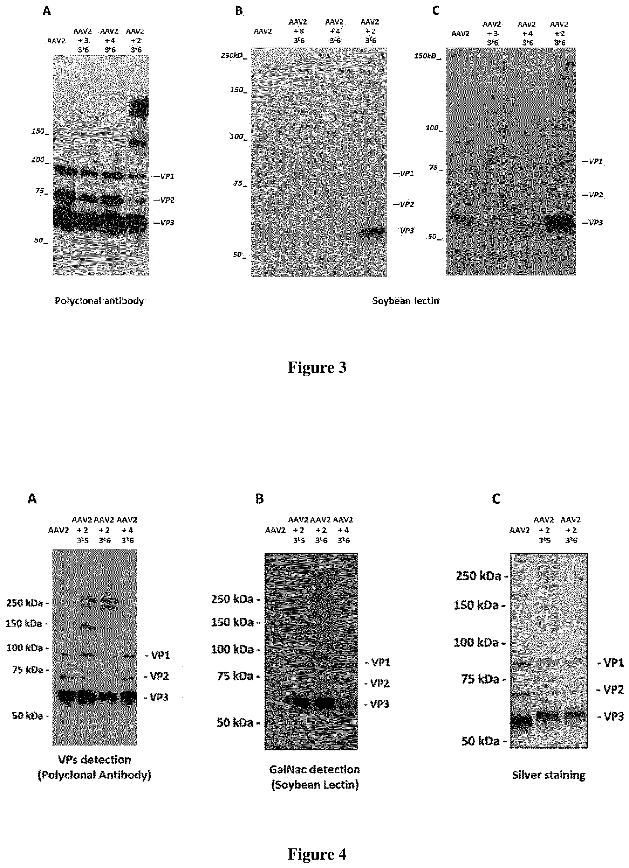

Figure 3, Figure 4: Western blots. 10¹² vg of AAV2-Luc-GFP or AAV2-GFP vectors were added to a solution of compounds **2, 3** or **4** (3E5 or 3E6 eq) in TBS buffer (pH 9.3) and incubated for 4 h at RT. 10¹² vg of the samples was analyzed by Western blot using a polyclonal antibody against the capsid proteins to detect VP proteins **(A)** or using a FITC-soybean lectin that recognizes the GalNAc sugar **(B-C).** VP1, VP2 and VP3 are the three proteins constituting the AAV capsid. Capsid protein molecular weight is indicated at the right of the images according to a protein ladder. B and C are images from the same blot with short (B) and long (C) exposure times during development.

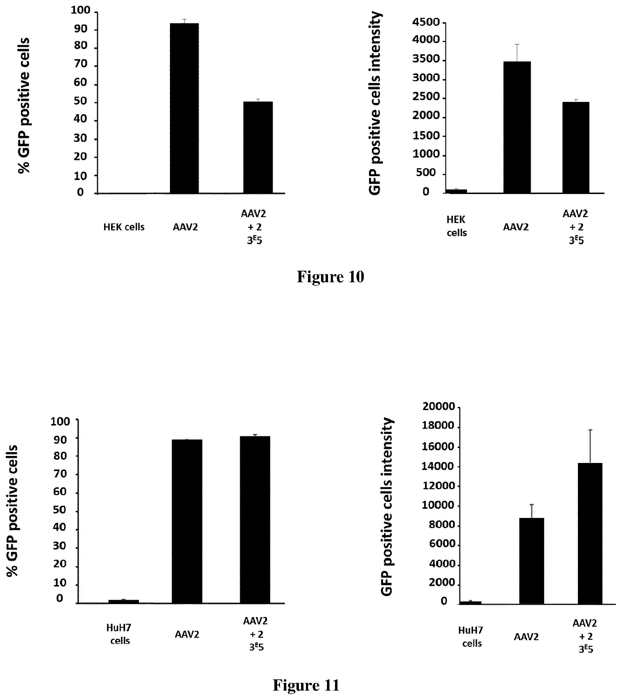

Figure 10: Infectivity of AAV2-GFP and AAV2 +2: AAV2 chemically modified with compound **2** (invention) (3E5 and 3E6 eq) on HEK cells by FACS analysis.
Figure 11: Infectivity of AAV2-GFP and AAV2 +2: AAV2 chemically modified with compound **2** (invention) (3E5 and 3E6 eq) on HuH7 cells by FACS analysis.

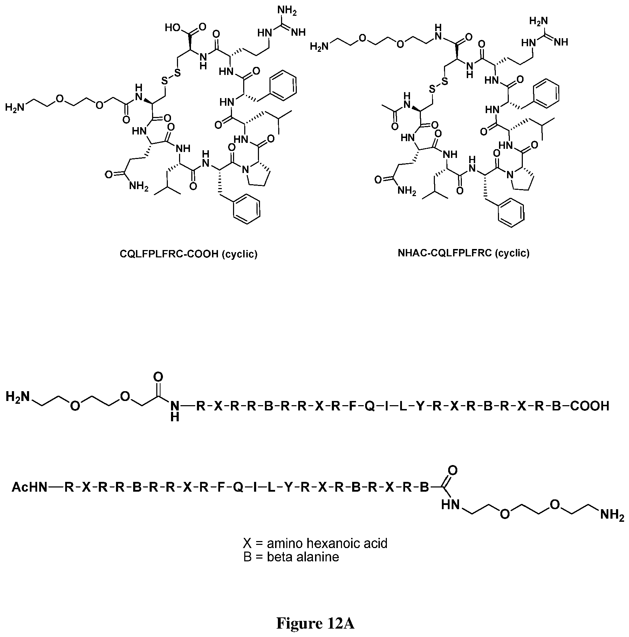

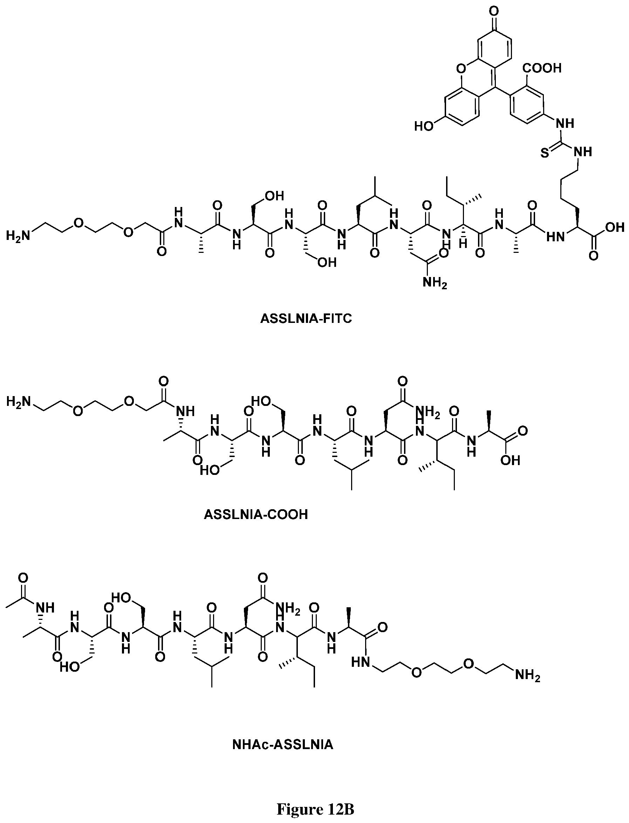

Figure 12A and 12B: show examples of "M" moieties according to the invention.

### Detailed description of the invention

The references to methods of treatment in the subsequent paragraphs of this description are to be interpreted as references to the compounds, pharmaceutical compositions and medicaments of the present invention for use in a method for treatment of the human body by therapy (or for diagnosis where appropriate).

To the best of the knowledge of the Inventors, the methods described in the prior art for chemically modifying AAV capsids target amino acid residues bearing amino groups such as arginine and lysine. However, there are other amino acid residues of interest to chemically modify such as surface-exposed tyrosine residues. Indeed, surface exposed tyrosine may play a pivotal role in in the immunogenicity as well as in the proteasome degradation AAV in cell. The Inventors have conceived a new method enabling to chemically modify tyrosine residues present on the surface of AAV capsid. This method relies on the specific reaction of an aryl diazonium group or PTAD group with the phenyl group in tyrosine residue resulting in covalent coupling.

The inventors prepared several PTAD and diazonium ligands bearing sugar moiety for chemically modifying the capsids of AAV vectors on tyrosine residues.

As a proof of concept, the Inventors showed that N-acetyl galactosamine and mannose moiety can be covalently immobilized on the surface of AAV capsid by incubating AAV particles with an aryl diazonium bearing N-acetyl galactosamine or mannose (compounds 2, 6,7 and 12) in aqueous buffer at basic pH, as evidenced by dot blot analysis with soybean and concanavalin A lectin detection.

The Inventor further showed that the covalent coupling did not alter capsid protein subunits VP1, VP2 and VP3 of AAV2 and AAV8, suggesting that the integrity of AAVs is maintained, as also evidenced by immunostaining with A20 antibody and ADK8 antibody respectively. Of note, the Inventors further showed that the AAV2 vectors chemically modified with the diazonium compound 2 still remain infectious in non-hepatic cell line HEK cells but display a transduction efficacy less important than that observed with non-chemically modified vectors (Fig 10). On the contrary on hepatic cell line HuH7 (that express low levels of ASPGR), AAV2 vectors chemically modified with the diazonium compound 2 showed an increased expression of the transgene evidenced by higher fluorescence intensity (Fig 11). Thus, the chemical modification of tyrosine residues of the capsid with sugar-bearing ligands would result in a retargeting of AAV and modification of their tropism in vivo, whereby off-target effects might be reduced.

### • Chemically-modified AAV of the invention

Accordingly, the invention relates to an Adeno-Associated Virus (AAV) having at least one chemically-modified tyrosine residue in its capsid.

As used herein, an Adeno-Associated Virus (AAV) refers to a small, nonenveloped virus of the *dependoparvovirus* family having a single-stranded linear DNA genome of about 5kb long. Wild-type AAV has two major open reading frames (ORFs) flanked by two inverted terminal repeats (ITRs). The 5' and 3' ORFs encode replication and capsid proteins, respectively. The ITR contains 145 nucleotides and serves as the AAV genome replication origin and packaging signal. In recombinant AAV, viral ORFs are replaced by the exogenous gene expression cassette, while the replication and capsid proteins are provided in *trans.*

Accordingly, in the context of the Invention, a recombinant AAV refers to an AAV wherein an exogenous nucleic acid sequence has been introduced in the viral genome. Said exogenous nucleic acid sequence may be of any type and is selected in view of the intended use of the AAV. For instance, said nucleic acid may comprises any RNA or DNA sequence.

In preferred embodiments, the AAV of the invention is a recombinant AAV. Typically, said recombinant AAV is to be used as a gene vector for *in vivo* or *in vitro* applications that means that the AAV of the invention is a recombinant AAV vector. For review concerning AAV as vector in gene therapy, one can refer to Naso et al., Biodrugs, 2017, 31:317-334.

For illustration only, a recombinant AAV for use as vector in gene therapy may comprise an exogenous gene expression cassette replacing the viral ORFs and placed between the two ITRs. Said cassette may comprise a promoter, the gene of interest and a terminator. The promoter and the gene of interest are selected depending on the targeted tissue/organ and the condition to treat. As another example, the recombinant AAV for use in gene therapy may comprise a DNA template for homologous recombination in cells. Such a recombinant AAV can be used in combination with gene editing tools, for promoting homologous recombination in targeted cells, *in vivo, in vitro* or *ex vivo.* The gene editing tools can be of any type, and encompass, without being limited to, CRISPR/Cas9, Zinc Finger Nuclease, meganuclease as well as RNA and DNA encoding said proteins.

In the context of the invention, the term "AAV" include all types of AAV, including wild-type AAV and recombinant or variant AAV. AAV variants encompass, without being limited to, AAV having a mutated or a synthetic capsid such as AAV with hybrid capsid, pseudotype AAV as well as self-complementary AAV (scAAV).

The capsid of a wildtype AAV is composed of three overlapping capsid proteins called viral protein 1 (VP1), VP2, and VP3. Genetic engineering of the capsid refers to amino acid modifications of said capsid protein(s), e.g. in their hypervariable loops.

As used herein, *"an AAV having a genetically engineered capsid'* or *"An AAV having a mutated capsid*" refers to an AAV wherein one or several amino acid modifications has(ve) been introduced in at least one capsid protein (namely VP1, and/or VP2 and/or VP3) as compared to the wild-type version of said capsid protein.

As used herein, "*an amino acid modification"* encompass the insertion, deletion or substitution of one or more (e.g. 1, 2, 3, 4, 5, 6, 7, 9, 10, 15, 20, 30, 40, 50, or 100) amino acids.

As used herein, "*a chemically-modified tyrosine residue"* means that at least one tyrosine present in the capsid of the virus has been chemically modified by covalent coupling of a chemical entity, typically by the covalent coupling of a said chemical entity on the phenyl ring of the tyrosine. Said tyrosine is typically a surface exposed residue present in VP1, VP2 or VP3. A surface exposed tyrosine means that the tyrosine is reachable for covalent coupling. Such tyrosine residues can be identified by molecular modelling of the capsid proteins or that of the whole capsid itself.

As used herein, *"at least one chemically-modified tyrosine residue"* encompasses at least 1, 2, 3, 4, 5, 6, 7, 8, 9 10, 20, 30, 40, 50, 60, 70, 80, 90, 100 or more *chemically-modified tyrosine residue(s).*

In some embodiments, the chemically-modified AAV of the invention comprises several chemically modified tyrosine residues in its capsid.

Said chemically-modified tyrosine may be present on VP1, and/or VP2 and/or VP3.

In some embodiments at least 0.1%, for instance at least 0.5, 1, 2, 3, 5, 10, 15, 20, 25, 30, 35, 40% or more of the surface exposed tyrosine residues of the capsid are chemically modified. In the context of the invention, the AAV may be either an AAV with wildtype capsid or an AAV having a mutated and/or a synthetic capsid.

In some embodiments, the AAV of the invention is a recombinant AAV with a wildtype capsid. In other embodiment, the AAV is a recombinant AAV having a mutated capsid, namely one or more amino acid modifications in at least one capsid protein as compared to the corresponding parent capsid protein.

In a particular embodiment, the AAV is a recombinant AAV having a mutated capsid, wherein the amino acid modification(s) is/are not concerned with any tyrosine residues present in capsid proteins.

They are various serotypes of AAV which can be either wildtype or synthetic. All serotypes are contemplated in the framework of the invention.

A "serotype" is traditionally defined on the basis of a lack of cross-reactivity between antibodies to one virus as compared to another virus. Such cross-reactivity differences are usually due to differences in capsid protein sequences/antigenic determinants (e.g., due to VP1, VP2, and/or VP3 sequence differences of AAV serotypes). AAV includes various naturally and synthetic (e.g. hybrid, chimera or shuffled serotypes) serotypes.

Such non-limiting serotypes include AAV-1, -2, -3, -4, -5, -6, -7, -8, -9, -10 (such as -cy10 or - rh10), -11, -rh74 or engineered AAV capsid variants such as AAV-2i8, AAV2G9, -LK3, -DJ, and -Anc80. In the context of the invention, synthetic serotypes also include pseudotyped AAV, namely AAV resulting from the mixing of a capsid and genome from different viral serotypes, such as AAV2/5, AAV2/7, and AAV2/8 as well as AAV with hybrid capsids derived from multiple different serotypes such as AAV-DJ, which contains a hybrid capsid derived from eight serotypes.

Synthetic serotypes also encompass specific variants wherein a new glycan binding site is introduced into the AAV capsid are in particular described in WO2014144229 (disclosing in particular the AAV2G9 serotype). Other AAV serotypes include those disclosed in EP2292779, and EP1310571. In addition, other AAV serotypes include those obtained by shuffling, as described in Koerber et al. (Molecular Therapy (2008), 16(10), 1703-1709), peptide insertion (e.g. Deverman et al., Nat Biotechnol (2016), 34(2), 204-209), or rational capsid design (reviewed in Büning et al., Curr Opin Pharmacol (2015), 24, 94-104).

In some embodiments, the AAV is selected from naturally-occurring serotypes, preferably from the group consisting of AAV-2, AAV-3b, AAV-5, AAV-8, AAV-9 and AAVrh10, more preferably AAV-2. For instance, the AAV of the invention may be a recombinant AAV vector of serotype -2.

The AAV can target a large variety of cells, tissues and organs. Examples of cells targeted by AAV encompasses, but are not limited to, hepatocytes; cells of the retina; i.e. photoreceptors, retinal pigmented epithelium (RPE),; muscle cells, i.e. myoblasts, satellite cells; cells of the central nervous system (CNS), i.e. neurons, glial; cells of the heart; cells of the peripheral nervous system (PNS); osteoblasts; tumor cells, blood cells such as lymphocytes, hematopoietic cells including hematopoietic stem cells, induced pluripotent stem cells (iPS) and the like.

Examples of tissues and organs which can be targeted by AAV include liver, muscle, cardiac muscle, smooth muscle, brain, bone, connective tissue, heart, kidney, lung, lymph node, mammary gland, myelin, prostate, testes, thymus, thyroid, trachea, and the like. Preferred cell types are hepatocytes, retinal cells, muscle cells, cells of the CNS, cells of the PNS and hematopoietic cells. Preferred tissue and organs are liver, muscle, heart, eye, and brain.

The tropism of AAV can vary depending on their serotype. For instance, AAV-2 can be used to transduce the central nervous system (CNS), kidney, and photoreceptor cells while AAV-8 is effective for transducing the CNS, heart, liver, photoreceptor cells, retinal pigment epithelium (RPE) and skeletal muscle.

The AAV can be produced by any methods known in the art, such as transient transfection in cell lines of interest e.g. in HEK293 cells as described in the Example section. To that matter one can refer to Naso et al., Biodrugs, 2017, 31:317-334 which provide a review on AAV as vectors in gene therapy, and describe the traditional methods for producing AAV at the industrial scale.

The AAV of the invention may have other amino acid(s) of the capsid which has been chemically modified. For instance, the AAV may comprise one or several amino groups of the capsid which have been modified by the method disclosed in WO2017/212019, namely by reacting said amino group(s) in the capsid with a ligand bearing an isothiocyanate reactive groups. Alternatively or additionally, the AAV of the invention may have one or several arginine residues of the capsid modified by glycation, e.g. by reaction with methylglyoxal as described in Horowitz (*supra*).

Typically, the at least one chemically-modified tyrosine residue in the capsid is of formula (I): 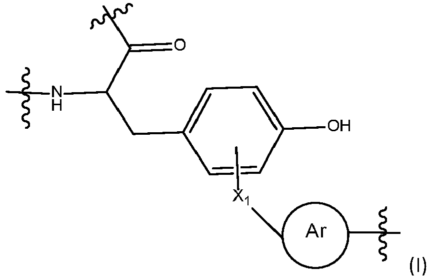 Wherein:
- X₁ is selected from the group consisting of: 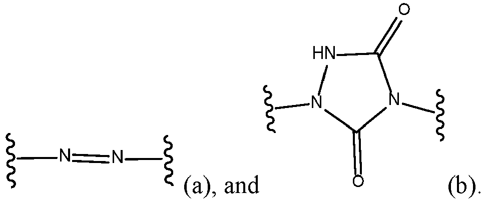 and
- Ar is an arylene or a heteroarylene moiety optionally substituted.

In a preferred embodiment, the AAV of the invention has at least one chemically-modified tyrosine residue in its capsid, wherein said at least one chemically-modified tyrosine residue is of formula (Ia): 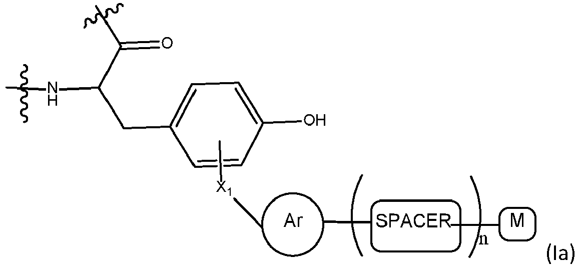 Wherein:
- X₁ is selected from the group consisting of: 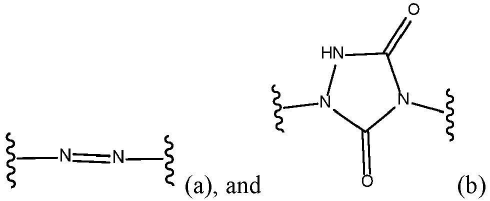
- n is 1 or 0. "n is 1" means that the group "spacer" is present, "n is 0" means that spacer is absent,
- Ar is an aryl or a heteroaryl moiety optionally substituted,
- Spacer is a chemical group which links "Ar" to "M". ,
- M is a functional moiety, the nature of which depending on the functional modification of the AAV which is sought by performing the chemical modification.
- *X₁*

X₁ is of formula (a), or (b) as described above. Preferably X₁ is of formula (a).

X₁ may be at position ortho, meta or para, preferably at position para.

### - Ar group

As mentioned above, Ar is a substituted or unsubstitued aryl or heteroaryl moiety.

As used herein, "*aryl*" refers to an aromatic ring system, which preferably has 6-14 atoms, having at least one ring having a conjugated pi electron system and which optionally may be substituted. An "*aryl*" may contain more than one aromatic ring such as fused ring systems or an aryl group substituted with another aryl group. Aryl encompass, without being limited to, phenyl, anthracenyl, naphthyl, indenyl, divalent biphenyl.

"*Heteroaryl*" refers to a heteroaryl group. "*Heteroaryl group*" refers to a chemical group, preferably having 5-14 ring atoms, wherein 1 to 4 heteroatoms are ring atoms in the aromatic ring and the remainder of the ring atoms being carbon atoms. Suitable heteroatoms include oxygen, sulfur, nitrogen, phosphorus, and selenium. Suitable heteroaryl groups include furanyl, thienyl, pyridyl, pyrrolyl, N-alkyl pyrrolyl, pyridyl-N-oxide, pyrimidyl, pyrazinyl, imidazolyl, benzimidazolyl, benzofuranyl, benzothiophenyl, quinazolinyl, and quinolinyl.

Examples of bicyclic heteroaryl groups encompass, without being limited to bicyclic heteroaryl groups that may be mentioned include 1H-indazolyl, benzo[l ,2,3]thiadiazolyl, benzo[l,2,5]thiadiazolyl, benzothiophenyl, imidazo[l,2-a]pyridyl, quinolinyl, indolyl and isoquinolinyl groups

"*Substituted*" or "*optionally substituted*" includes groups substituted by one or several substituents, typically 1, 2, 3, 4, 5 or 6 substituents. For instance, the substituents may be independently selected from C₁-C₆ alkyl, aryl group, C₃-C₆ cycloalkyl, C₂-C₆ heterocycloalkyl, C₁-C₆ alkoxy, C₁-C₆ alkylamino, C₁-C₆ aminoalkyl-, C₁-C₆ alkylaminoalkyl-, -N₃, -NH₂, -F, - I, -Br, -Cl, -CN, C₁-C₆ alkanoyl, C₁-C₆ carboxy esters, C₁-C₆ acylamino, -COOH, -CONH₂, - NO₂, -SO₃H, C₁-C₆ aminoalkyl, C₁-C₆ hydroxyalkyl, C₁-C₆ haloalkyl, C₁-C₆ alkylthio, C₂-C₁₀ alkoxyalkyl, C₂-C₆ alkoxycarbonyloxy, -CN, -CF₃ and C₂-C₆ alkoxyalkyl.

Preferred substituents are halogens, -OH, NH₂, NO₂, C₁-C₃ alkyl, C₁-C₃ alkoxy, C₁-C₃ hydroxyalkyl, and C₁-C₃ haloalkyl.

The phrase "*optionally substituted*" can be replaced by the phrase "*substitutedor unsubstituted*" throughout this application.

In some embodiments, "Ar" is selected from aryl and hereoaryl comprising from 5 to 14 ring atoms, e.g. from 6 to 10 ring atoms, said aryl or heteroaryl being optionally substituted. For instance, said aryl or heteroaryl group may comprise 1, 2 or 3 substituents independently selected from halogens, -OH, NH₂, NO₂, C₁-C₃ alkyl, C₁-C₃ alkoxy, C₁-C₃ hydroxyalkyl, and C₁-C₃ haloalkyl.

In some particular embodiments, "Ar" is selected from the group consisting of substituted or unsubstituted phenyl, pyridyl, naphthyl, and anthracenyl. Said aromatic compounds may comprise from 1 to 3 substituents, preferably independently selected from halogens, -OH, NH₂, NO₂, C₁-C₃ alkyl, C₁-C₃ alkoxy, C₁-C₃ hydroxyalkyl, and C₁-C₃ haloalkyl.

### - Spacer group

"Spacer" is an optional chemical group, which can be thus present or absent.

When present, "Spacer" is used to link the "Ar" group to the functional moiety "M". "Spacer" may be any chemical chain which can comprise heteroatoms as well as cyclic moieties such as cycloalkyl or aromatic groups. "Spacer" may comprise up to 1000 carbon atoms and even more. The length and the chemical nature of the spacer may be optimized depending on the "M" moiety which is intended to be coupled with the tyrosine residues and the biological effect which is sought. Indeed, further to its linking function, "Spacer" may be used to refine the properties of the functional moiety "M". For instance, "Spacer" may decrease the steric hindrance of "M" with respect to the capsid, improve the accessibility of "M" for binding with a biological entity of interest, improve the binding of "M" with an entity of interest and/or increase the solubility of "M".

In some embodiments, "Spacer" is a chemical chain group comprising from 2 to 1000 carbon atoms, preferably from 2 to 500 carbon atoms, from 2 to 300 carbon atoms, e.g. from 2 to 100 carbon atoms, 2 to 40 carbon atoms, from 4 to 30 carbon atoms or from 4 to 20 carbon atoms.. Typically, "Spacer" is selected from the group consisting of polymers including homopolymers, copolymers and block polymers, peptides, oligosaccharides, and a saturated or unsaturated hydrocarbon chains optionally interrupted by one or several heteroatoms (e.g. S, O, Se, P or NH), optionally having at least one of its extremity an heteroatom such as S, O and NH, and optionally substituted by one or several substituents such as hydroxyl, halogens, C₁-C₃ alkoxy, -CN, -CF₃, or C₁-C₃ alkyl, and combinations thereof.

As used herein, "*combinations*" means that the spacer group may comprise several hydrocarbon chains, oligomer chains or polymeric chains (e.g. 2, 3, 4, 5 or 6) linked by any appropriate group, such as -O-, -S-, -NHC(O)-, -OC(O)-, -C(O)-O-C(O)-, -NH-, -NH-CO-NH-, -O-CO-NH-, NH-(CS)-NH-, NH-CS- phosphodiester or phosphorothioate groups.

In some embodiments, the spacer may be selected from the group consisting of polyethers such as polyethylene glycol (PEG) and polypropylene glycol, polyvinyl alcohol (PVA), polyesters such as polylactate, polyacrylate, polymethacrylate, polysilicone, polyamide such as polycaprolactone and poly(N-(2-hydroxypropyl)methacrylamide) (pHPMA), poly(D,L-lactic-co-glycolic acid) (PLGA), polymers of alkyl diamines, unsaturated or saturated, branched or unbranched, hydrocarbon chains optionally having an heteroatom such as O, NH and S on at least one end, and combinations thereof.

As used herein, alkyl diamine refers to NH₂-(CH₂)ᵣ-NH₂ with r is an integer from 2 to 20, for instance from 2 to 10 such as 2, 3, 4, and 5. A polymer of alkyl diamines (also known as polyamines) refers to a compound of formula NH₂-[(CH₂)ᵣ-NH]ₜ-H with r being as defined above and t is an integer of at least 2, for example of at least 3, 4, 5, 10 or more. Polymers of alkyl diamines of interest are, for instance, spermidine, and spermine

For instance, the spacer comprises at least one polyethylene glycol moiety comprising from 2 to 40 monomers, e.g. from 2 to 10 or 2 to 6 monomers. For illustration only, the spacer may comprise from 2 to 10 triethyleneglycol blocks linked together by linkers. As another example, the spacer may be a C₁₂ hydrophilic triethylene glycol ethylamine derivative. Alternatively, the spacer may be a saturated or unsaturated C₂-C₄₀ hydrocarbon chain, in particular a C₁₀-C₂₀ alkyl chain or a C₂-C₁₀ alkyl chain such as a C₆ alkyl chain. The alkyl chain may have a group such as NH, S or O on at least one end.

As further examples, the spacer may be selected from spermidine, putrescine, spermine and combinations thereof.

In a particular embodiment, the spacer group is selected from the group consisting of linear or branched C₂-C₂₀ alkyl chains, polyethylene glycol, polypropylene glycol, pHPMA, PLGA, polymers of diamino alkyl and combinations thereof. Preferably said polyethylene glycol, polypropylene glycol, PLGA,pHPMA and polymer of alkyl diamines comprise from 2 to 40 monomers, preferably from 2 to 10 or from 10 to 20 monomers.

For instance, the group "spacer" may comprise one or several (e.g. 2, 3, 4 or 5) triethyelene glycol blocks.

### - "M" moiety

The functional moiety "M" may be of any type. "M" is typically selected depending on the biological effect which is sought when chemically modifying the capsid of the AAV.

For instance, "M" may comprise a moiety selected from a targeting agent, a steric shielding agent, a labelling agent, or a drug. "M" may be also a (nano)-particle, including a magnetic (nano-) particle and a quantum dot. For instance M may be an iron, stain, silicium, gold or carbon (nano)-particle.

In some embodiments, "M" comprises, or consists of, a labeling agent, e.g. a fluorescent dye such as fluorescein, rhodamine, boron-dipyrromethene (Bodipy) dyes, and alexa fluor, or a radionuclide.

In other embodiments, "M" comprises, or consists of, a steric shielding agent, e.g. an agent able to mask certain epitopes of the capsid, whereby avoiding the binding of neutralizing antibodies. For instance, "M" may be a polyethylene glycol (PEG), pHPMA or a polysaccharide.

In a specific embodiment, "M" comprises, or consists of, a steric shielding agent able to mask tyrosine residues, whereby proteasome-degradation of the AAV in cellulo is avoided.

In some embodiments, M comprises, or consists of, a cell-type specific ligand, namely a ligand enabling to target a specific type of cell. Such a ligand may enable to modify the tropism of the AAV, namely its capacity to selectively infect and/or transduce a given cell line, tissue or organ. For instance, "M" may be a ligand which specifically binds to a membrane biological entity (e.g. a membrane receptor) of the targeted cell. Said ligand may be, for instance, a mono- or a polysaccharide, a hormone, including a steroid hormone, a peptide such as RGD peptide, Angiopep-2, muscle targeting peptides , a protein or a fragment thereof, a membrane receptor or a fragment thereof, CB1 and CB2 ligands, an aptamer, an antibody including heavy-chain antibody, and fragments thereof such as Fab, Fab', and VHH, a ScFv, a spiegelmer, a peptide aptamer, a small chemical molecules known to bind to the targeted biological entity and the likes.

In some embodiments, "M" comprises, or consists of, a cell-type specific ligand derived from proteins such as transferrin, Epidermal Growth Factor (EGF), and basic Fibroblast Growth Factor βFGF.

In some other embodiments, "M" comprises, or consists of, a cell-type specific ligand derived from mono- or polysaccharides, e.g. comprising one or several galactose, mannose, mannose-6-phosphate, N-acetylgalactosamine (GalNac) and bridged GalNac. The mono- or polysaccharides can be natural or synthetic.

In another embodiment, "M" comprises, or consists of, a cell-type specific ligand derived from vitamins such as folic acid.

According to one embodiment, the cell-type specific ligand included in "M" may be derived from, or may consist in, a muscle targeting peptide (MTP). Said ligand may comprise an amino acid sequence selected from the group consisting of: ASSLNIA (SEQ ID NO: 1); WDANGKT (SEQ ID NO: 2); GETRAPL (SEQ ID NO: 3); CGHHPVYAC (SEQ ID NO: 4); HAIYPRH (SEQ ID NO: 5), cyclic CQLFPLFRC (SEQ ID NO: 6) or the sequence of SEQ ID NO:7 as shown below:
**R-X-R-R-B-R-R-X-R-F-Q-I-L-Y-R-X-R-B-R-X-R-B** (SEQ ID NO:6)
wherein X is an amino hexanoic acid residue and B is a beta-alanine residue.

As used herein, cyclic CQLFPLFRC of SEQ ID NO:6 refers to:

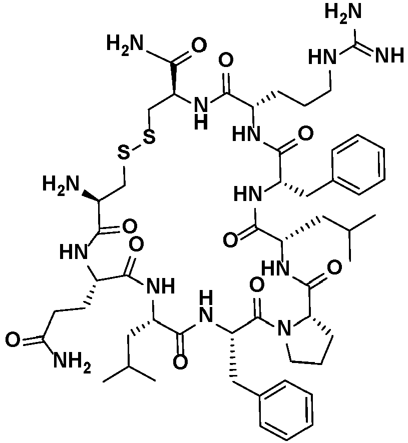

In certain embodiments, "M" is a cancer cell targeting peptide and comprises a peptide such as RGD, including cyclic RGD.

When "M" comprises a peptide moiety, such as a muscle targeting peptide (MTP), said peptide moiety may comprise a chemical modification at its N-terminus or C-terminus. For instance, the N-terminus of the peptide moiety can be acylated or coupled to a moiety such as -C(=O)-(PEG moiety)-NH₂.

In another embodiments, "M" comprises, or consists of, a cell-type specific ligand derived from small molecules or hormones such as naproxen, ibuprofen, cholesterol, progesterone or estradiol.

In an additional embodiment, "M" comprises, or consists of, a CB1 and/or a CB2 ligand, for instance:

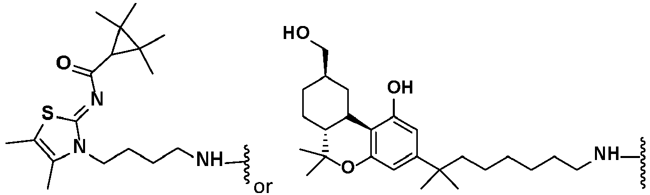

Galactose- derived ligands, which are recognized by asialoglycoprotein receptor (ASPGPr), can be used to specifically target hepatocytes. Accordingly, in some embodiments "M" is a ligand for specifically targeting hepatocytes and comprises at least one moiety of formula (IIIa), (IIIb) or (IIIc):

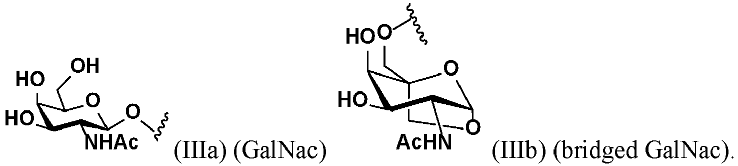

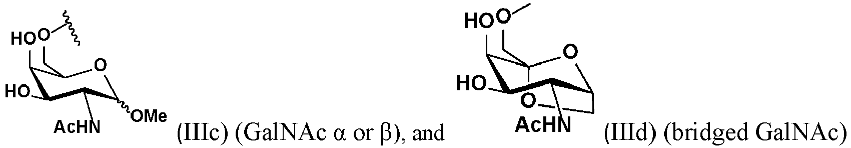

In some other embodiments, "M" is a ligand for targeting muscle cells, in particular skeletal muscle cells and comprises at least one mannose-6-phosphate moiety:

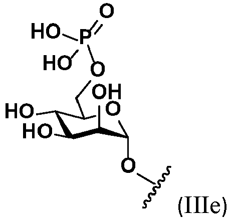

In some other embodiments, "M" is a ligand for photoreceptors or neuronal cells and comprises at least one mannose moiety of formula (IIIf):

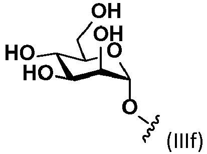

In some embodiments, "M" is multivalent, which means that it comprises at least two (e.g. 2, 3, 4, 5, or 6) ligand moieties of interest, such as cell-type specific ligands as described above. For instance, M may comprise a polyfunctional linker bearing several (e.g. at least 2, 3, 4, 5, or 6) cell-type ligands. The cell-type ligands can be the same or different.

For instance, "M" may comprise a moiety of formula (IV): 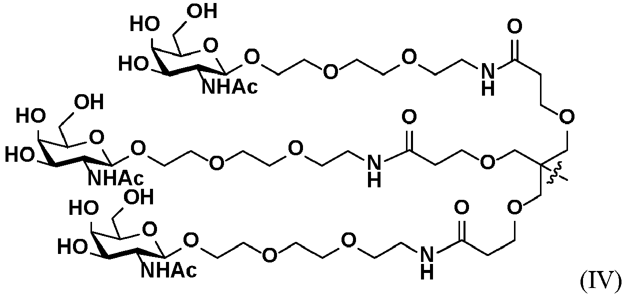 with n is a enter from 1 to 100, preferably from 1 to 20.

As another example of multivalent ligands, "M" may comprise a moiety of formula (IV) wherein the GalNac groups are replaced by mannose, phosphate-6-mannose,bridged GalNac, CB1 and/or CB2 ligands or peptides.

In some particular embodiment, "M" may comprise both a labelling moiety such as a fluorescent label or a radionuclide and a cell-type specific ligand. For illustration only, M may be: 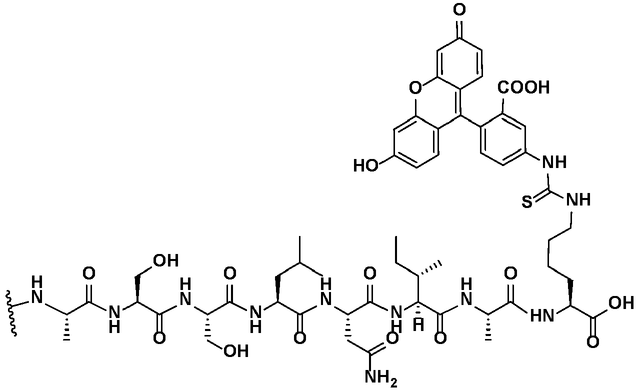 namely a muscle targeting peptide of SEQ ID NO: 1 linked to K-FITC.

Other examples of chemical moieties which can be used as "M" moieties are provided in Figure 12A and in Figure 12B.

As mentioned above, preferred chemically-modified AAV are those comprising at least one chemically-modified tyrosine of formula (Ia) or (I), wherein X₁ is of formula (a).

In a particular embodiment, X₁ is at position ortho of the phenyl group of the tyrosine. Accordingly, the invention relates to AAV particle comprising at least one chemically-modified tyrosine present in the capsid, which is of formula (Ib):

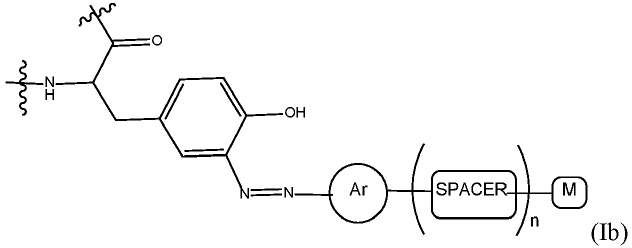

Wherein "Ar", "Spacer", n, and "M" are as defined above for formula (I).

In some embodiments of the invention, the chemically-modified tyrosine present in the capsid is of formula (Ib) and is further characterized by one or several of the following features:
- "Ar" is selected from the group consisting of substituted or unsubstituted phenyl, pyridyl, naphthyl, and anthracenyl. "Ar" may comprise from 1 to 3 substituents, preferably independently selected from halogens, -OH, NH₂, NO₂, C₁-C₃ alkyl, C₁-C₃ alkoxy, C₁-C₃ hydroxyalkyl, and C₁-C₃ haloalkyl, and/or
- "Spacer", when present, is selected from the group consisting of saturated or unsaturated, linear or branched C₂-C₄₀ hydrocarbon chains, optionally substituted, polyethylene glycol, polypropylene glycol, pHPMA, PLGA, polymers of alkyl diamines and combinations thereof, the polymers preferably comprising from 2 to 40 monomers, and/or
- "M" comprises, or consists of a cell-type targeting ligand, preferably selected from a mono- or a polysaccharide, a hormone, including a steroid hormone, a peptide such as RGD peptide, muscle targeting peptides (MTP), or Angiopep-2, a protein or a fragment thereof, a membrane receptor or a fragment thereof, an aptamer, an antibody including heavy-chain antibody, and fragments thereof such as Fab, Fab', and VHH, a ScFv, a spiegelmer, a peptide aptamer, a vitamin and small chemical molecules such as drugs e.g. CB1 and/or CB2 ligands.

In another embodiments, the chemically-modified tyrosine present in the capsid is of formula (Ib) and is further characterized by one or several of the following features:
- "Ar" is selected from a phenyl or a pyridyl optionally substituted, and/or
- "Spacer" (when present) is selected from the group consisting of linear or branched C₂-C₄₀ preferably C₂-C₂₀ alkyl chains, polyethylene glycol, polypropylene glycol, pHPMA, PLGA, polymers of alkyl diamines and combinations thereof, wherein the polymer preferably comprises from 2 to 40 monomers, and/or
- "M" comprises, or consists of, a cell-type specific ligand derived from proteins such as transferrin, Epidermal Growth Factor (EGF), and basic Fibroblast Growth Factor βFGF, muscle targeting peptides as described above and from mono- or polysaccharides, e.g. comprising one or several galactose, mannose, mannose-6-phosphate, N-acetylgalactosamine, or bridge GalNac, CB1 and/or CB2 ligands and vitamins such as folic acid.

In a particular embodiment, "Ar" is an optionally substituted phenyl. Typically, the phenyl can be substituted by one or several substituents selected from halogens such as Cl, Br, I, or F, C₁-C₆ alkyl,C₁-C₆ hydroxyalkyl, C₁-C₆ alkoxy, C₁-C₆ haloalkyl, -CN, -NO₂, and the like.

In some embodiments, "Ar" is an unsubstituted phenyl, said phenyl being linked to the SPACER group (when n is 1) or to the M group (when n is 0) by a linker X₂.

Accordingly, a further object of the invention is an AAV comprising at least one chemically-modified tyrosine, said chemically-modified tyrosine being of formula (Ic): 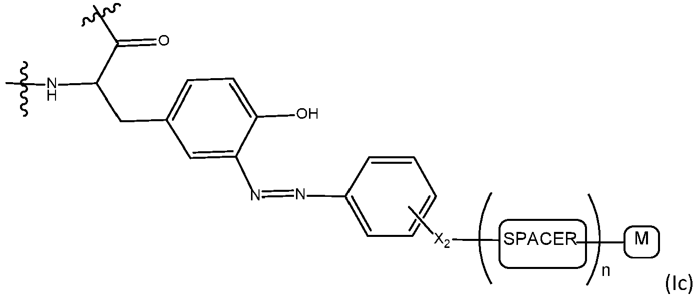 wherein:
- X₂ is -C(=O)-NH, -C(=O)-O, -C(=O)-O-C(=O)-, O-(C=O)-, NH-C(=O)-, NH-C(=O)-NH, and -O-C=O-O-, O, NH, NH(C=S)-, -(C=S)-NH-, preferably -(C=O)-NH- or -(C=O)-O]- and
- "Spacer", n and M being as described above
- X₂ may be at position para, meta or ortho, preferably at position para of the phenyl group. In some embodiments of the invention, the chemically-modified tyrosine present in the capsid is of formula (Ic) and is further characterized by one or several of the following features:
- "Spacer", when present, is selected from the group consisting of saturated or unsaturated, linear or branched C₂-C₄₀ hydrocarbon chains, optionally substituted, polyethylene glycol, polypropylene glycol, pHPMA, PLGA, polymers of alkyl diamines and combinations thereof, the polymers preferably comprising from 2 to 40 monomers, and/or
- "M" comprises, or consists of a cell-type targeting ligand, preferably selected from a mono- or a polysaccharide, a hormone, including a steroid hormone, a peptide such as RGD peptide, muscle targeting peptides (MTP), or Angiopep-2, a protein or a fragment thereof, a membrane receptor or a fragment thereof, an aptamer, an antibody including heavy-chain antibody, and fragments thereof such as Fab, Fab', and VHH, a ScFv, a spiegelmer, a peptide aptamer, a vitamin and small chemical molecules such as drugs e.g. CB1 and/or CB2 ligands.

In another embodiment, the chemically-modified tyrosine present in the capsid is of formula (Ic) and is further characterized by one or several of the following features:
- "Spacer" (when present) is selected from the group consisting of linear or branched C₂-C₄₀ preferably C₂-C₂₀ alkyl chains, polyethylene glycol, polypropylene glycol, pHPMA, PLGA, polymers of alkyl diamines and combinations thereof, wherein the polymer preferably comprises from 2 to 40 monomers, and/or
- "M" comprises, or consists of, a cell-type specific ligand derived from proteins such as transferrin, Epidermal Growth Factor (EGF), and basic Fibroblast Growth Factor βFGF, muscle targeting peptides as described above and from mono- or polysaccharides, e.g. comprising one or several galactose, mannose, mannose-6-phosphate, N-acetylgalactosamine, or bridge GalNac, CB1 and/or CB2 ligands and vitamins such as folic acid.

In some embodiments, X₂ is -C(=O)NH- and is at position para. Accordingly, the invention also relates to an AAV comprising at least one chemically-modified tyrosine present in its capsid, said chemically-modified tyrosine being of formula (Id):

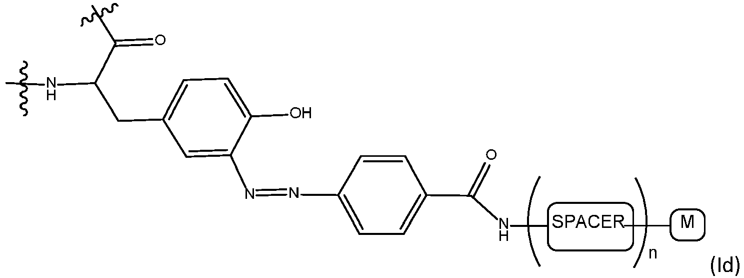

Wherein n, M and Spacer are as defined above.

In some embodiments n is 1.

In some further or other embodiments, the chemically-modified tyrosine present in the capsid is of formula (Id) and is further characterized by one or several of the following features:
- *"Spacer",* when present, is selected from the group consisting of saturated or unsaturated, linear or branched C₂-C₄₀ hydrocarbon chains, optionally substituted, polyethylene glycol, polypropylene glycol, pHPMA, PLGA, polymers of alkyl diamines and combinations thereof, the polymers preferably comprising from 2 to 40 monomers, and/or
- "M" comprises, or consists of a cell-type targeting ligand, preferably selected from a mono- or a polysaccharide, a hormone, including a steroid hormone, a peptide such as RGD peptide, muscle targeting peptides (MTP), or Angiopep-2, a protein or a fragment thereof, a membrane receptor or a fragment thereof, an aptamer, an antibody including heavy-chain antibody, and fragments thereof such as Fab, Fab', and VHH, a ScFv, a spiegelmer, a peptide aptamer, a vitamin and small chemical molecules such as drugs e.g. CB1 and/or CB2 ligands.

In another embodiments, the chemically-modified tyrosine present in the capsid is of formula (Id) and is further characterized by one or several of the following features:
- *"Spacer"* (when present) is selected from the group consisting of linear or branched C₂-C₄₀ preferably C₂-C₂₀ alkyl chains, polyethylene glycol, polypropylene glycol, pHPMA, PLGA, polymers of alkyl diamines and combinations thereof, wherein the polymer preferably comprises from 2 to 40 monomers, and/or
- "M" comprises, or consists of, a cell-type specific ligand derived from proteins such as transferrin, Epidermal Growth Factor (EGF), and basic Fibroblast Growth Factor βFGF, muscle targeting peptides as described above and from mono- or polysaccharides, e.g. comprising one or several galactose, mannose, mannose-6-phosphate, N-acetylgalactosamine, or bridge GalNac, CB1 and/or CB2 ligands and vitamins such as folic acid.

In some other embodiments, the chemically-modified tyrosine present in the capsid is of formula (Ia) with X₁ is of formula (b), preferably at position ortho and is further characterized by one or several of the following features:
- *"Spacer",* when present, is selected from the group consisting of saturated or unsaturated, linear or branched C₂-C₄₀ hydrocarbon chains, optionally substituted, polyethylene glycol, polypropylene glycol, pHPMA, PLGA, polymers of alkyl diamines and combinations thereof, the polymers preferably comprising from 2 to 40 monomers, and/or
- "M" comprises, or consists of a cell-type targeting ligand, preferably selected from a mono- or a polysaccharide, a hormone, including a steroid hormone, a peptide such as RGD peptide, muscle targeting peptides (MTP), or Angiopep-2, a protein or a fragment thereof, a membrane receptor or a fragment thereof, an aptamer, an antibody including heavy-chain antibody, and fragments thereof such as Fab, Fab', and VHH, a ScFv, a spiegelmer, a peptide aptamer, a vitamin and small chemical molecules such as drugs e.g. CB1 and/or CB2 ligands.

In another embodiments, the chemically-modified tyrosine present in the capsid is of formula (Ia) with X₁ is of formula (b), preferably at position ortho, and is further characterized by one or several of the following features:
- *"Spacer"* (when present) is selected from the group consisting of linear or branched C₂-C₄₀ preferably C₂-C₂₀ alkyl chains, polyethylene glycol, polypropylene glycol, pHPMA, PLGA, polymers of alkyl diamines and combinations thereof, wherein the polymer preferably comprises from 2 to 40 monomers, and/or
- "M" comprises, or consists of, a cell-type specific ligand derived from proteins such as transferrin, Epidermal Growth Factor (EGF), and basic Fibroblast Growth Factor βFGF, muscle targeting peptides as described above and from mono- or polysaccharides, e.g. comprising one or several galactose, mannose, mannose-6-phosphate, N-acetylgalactosamine, or bridge GalNac, CB1 and/or CB2 ligands and vitamins such as folic acid.

For illustration only, the AAV of the invention may comprise at least one chemically-modified tyrosine in the capsid, of formula (IIa), (IIb), (IIc) or (IId):

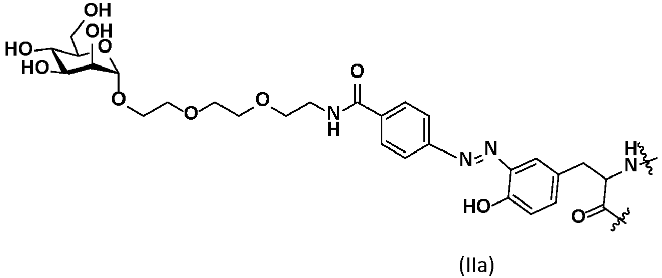

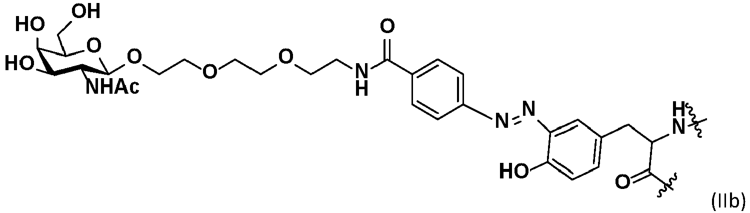

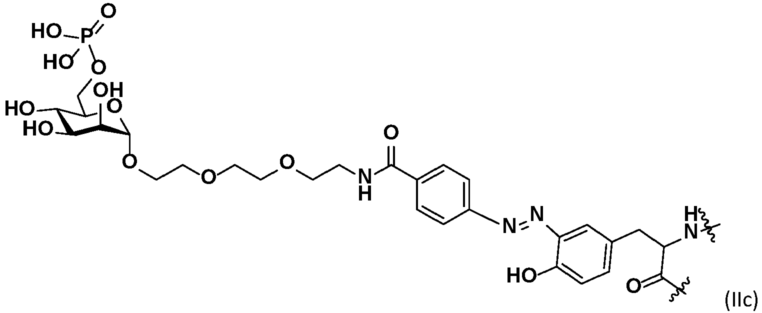

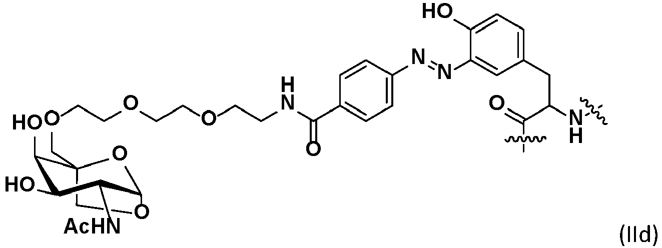

The AAV of the invention may comprise at least one chemically-modified tyrosine in the capsid, of formula (IIe), (IIf), or (IIg): 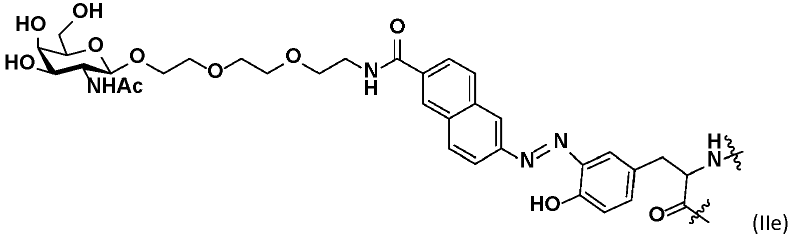 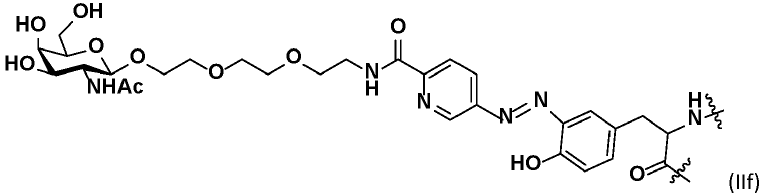 and

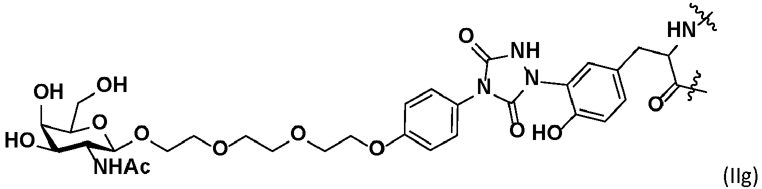

In all the embodiments described above, especially wherein the at least one chemically-modified protein is of formula (I), (Ia), (Ib), (Ic) (Id),(IIa), (IIb), (IIc) (IId), (IIe), (IIf) and (IIg), the AAV is preferably a recombinant AVV, more preferably a recombinant AAV vector.

As mentioned above, the AAV may have a "naturally-occurring" capsid or a genetically modified capsid, namely comprising one or several mutations in at least one capsid protein, namely VP1, VP2 and/or VP3.

In some additional or alternate embodiments, the AAV may be of a serotype selected from AAV-2, AAV-3b, AAV-5, AAV-8, AAV-9 and AAVrh10, more preferably AAV-2. Alternatively, the AAV is of a synthetic serotype.

In some further embodiments, the AAV of the invention may have at least one additional chemically modified amino acid residue in the capsid, which is different from a tyrosine residue, e.g. an arginine or a lysine residues. In some embodiments, said amino acid residue bears an amino group in its side chain and is chemically modified with a group of formula (V):

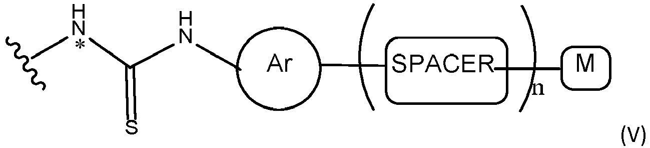

Wherein:
- N* being the nitrogen of the amino group of the side chain of an amino acid residue, e.g of a lysine residue,
- Ar, Spacer, n and M being as defined in Formula (Ia), (Ib), (Ic) and (Id). It goes without saying that Ar, Spacer, n and M in formula (V) can be the same or different as those present in the at least one chemically-modified tyrosine as described above.

Said modification on the amino group can be introduced as described in WO2017212019.

The chemical modification(s) of the capsid of the AAV may modify one or several biological functionalities and/or properties. Depending on the nature of "M" which is covalently bound on the surface of the chemically-modified AAV, said chemically-modified AAV may have one or several modified biological properties as compared to the same but non-chemically modified AAV, such as:
- A modified tropism e.g. an increased selectivity of the AAV towards a specific organ, tissue or cell (either administered in vivo or transducing tissues or cells in culture) or a shifted selectivity of the AAV from one tissue/organ/cell to another, and/or
- An altered immunoreactivity of the AAV, e.g. a decreased immunogenicity of the AAV and/or a decreased affinity for neutralizing antibodies, and/or said AAV triggers an altered humoral response when administered in vivo, e.g. do not generate AAV-directed neutralizing antibodies
- An increased infectivity efficiency of the AAV particles, and/or
- An increased transduction efficacy of the AAV towards a specific cell, tissue or organ.
- A reduced cellular toxicity when transducing cells in culture
- Induce cellular targeted mortality into cancer cells
- Visualization/monitoring of the AAV particle upon in vivo administration or upon modification of cells in vitro with these AAV particles
- Theragnostic applications; e.g. combining a therapeutic agent and a diagnostic agent

In some embodiments, the chemically-modified AAV of the invention may have a higher transduction efficiency, which may result from increased intracellular trafficking to the nuclei, a decrease in proteasome-degradation, more efficient intranuclear de-capsidation, more rapid vector genome stabilization and/or from a decrease in interaction with neutralizing antibodies and/or from a reduction of antibody-mediated clearance of AAV in vivo, as compared to the non-chemically modified AAV. In some other embodiments, the AAV may have a higher infectivity efficiency and/or an increase in selectivity for a given cell, tissue or organ as compared to the non-chemically modified AAV either *in vivo* or *in vitro.*

In some other embodiments, when the AAV is used as a drug, e.g. as a gene vector, such modified properties may result in an improvement in the therapeutic index of the AAV, which may result from decrease in the dose to administer to the patient to achieve the sought therapeutic effect and/or a decrease in the toxicity of the AAV.

In a particular embodiment, the chemically-modified AAV of the invention shows a preferential tropism for an organ or cell selected from liver, heart, brain, joints, retina and skeletal muscle. In another or additional embodiment, the chemically-modified AAV of the invention shows a preferential tropism for cultured cells selected from, but not limited to, hepatocytes, cardiomyocytes, myocytes, neurons, motor neurons, retinal pigmented cells, photoreceptors, chondrocytes, hematopoietic stem cells (HSC) or induced pluripotent stem cells (iPS).

### • Methods for preparing the chemically-modified AAV of the invention

The invention also relates to a method for chemically-modifying the capsid of an AAV, more precisely for chemically modifying at least one tyrosine residue in the capsid of an AAV, which comprises incubating said AAV with a chemical reagent bearing a reactive group selected from an aryl diazonium salt, and a 4-phenyl-1,2,4-triazole-3,5-dione (PTAD) moiety in conditions conducive for reacting said reactive group with a tyrosine residue present in the capsid of the AAV so as to form a covalent bound.

In a particular embodiment, the method of the invention is for obtaining a chemically-modified AAV comprising at least one chemically-modified tyrosine residue in its capsid, wherein said chemically-modified tyrosine residue is of formula (Ia): 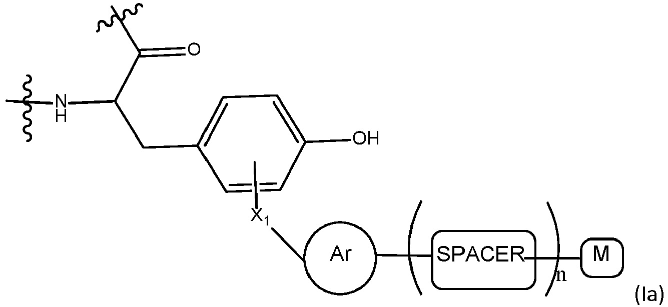 which comprises incubating the AAV with a chemical reagent selected from 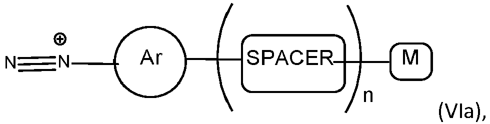 and 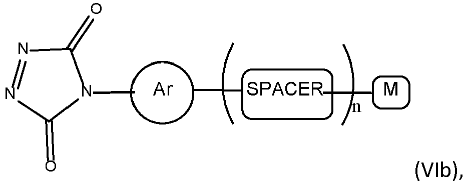 in conditions conducive for reacting said chemical reagent with a tyrosine residue present in the capsid of the AAV so as to form a covalent bound.

It goes without saying that:
- Ar, spacer, and n are as defined above for formula (Ia) and,
- when X₁ is of formula (a), the chemical reagent is of formula (VIa), and when X₁ is of formula (b), the chemical reagent is of formula (VIb).

Typically, the AAV particles are incubated with the chemical reagent in conditions suitable to promote the formation of a covalent bond between the phenyl group of a tyrosine residue and said chemical reagent without impairing the structural integrity of said AAV.

The incubation may be performed in an aqueous buffer having a pH from 5 to 11, preferably from 7 to 10, e.g. from 8.5 to 10.5 or. from 9 to 10.

The buffer may be selected from TRIS buffer, sodium carbonate - sodium bicarbonate buffer, phosphate buffer e.g. PBS or Dulbecco's phosphate-buffered saline (dPBS).

When the chemical reagent is of formula (VIa), the pH of the aqueous buffer may be basic, e.g. above 7 such as from 8.5 to 10, for instance from 9 to 10, e.g. from 9.0 to 9.6. Such a basic pH may enable to increase reactivity and/or specificity of the aryl diazonium salt reagent towards the phenyl group of tyrosine residue. For instance, an appropriate buffer to perform the incubation of the AAV with an aryldiazonium reagent is TRIS buffer.

The incubation may last from several minutes to several hours, for instance from 5 min to 6h, e.g. from 3 to 4 hours. Typically, the incubation is ended when a sufficient yield of coupling is achieved.

The temperature of incubation is typically from 10°C to 50°C. Preferably the incubation is performed at room temperature, i.e. at a temperature from 18°C to 30°C, e.g. at around 20°C. The incubation may be performed under stirring.

The molar ratio of the chemical reagent to the AAV particles may be from 1.10 to 1.10⁸, for instance from 1.10⁵ to 1.10⁷, e.g. 1.10⁶ to 5.10⁶.

The medium can comprise one or several additional compounds. For instance, when the chemical reagent is of formula (VIc), the medium may comprise an oxidant, or a system producing an oxidant, such as H₂O₂.

The counter-anion present in the aryl diazonium salt reagent can be of any type preferably TsO⁻ BF₄⁻, Cl⁻, AcO⁻, PF₆⁻, TfO⁻ or CF₃CO₂⁻

In a particular embodiment, the resulting AAV comprises at least one chemically-modified tyrosine of formula (Ib). Accordingly, the chemical reagent is of formula (VIa): 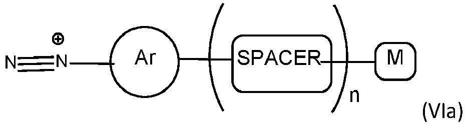 wherein Ar, spacer, n and M are as defined in formula (Ib).

In some other embodiments, the resulting AAV comprises at least one chemically-modified tyrosine of formula (Ic). Accordingly, the chemical reagent is of formula (VId):

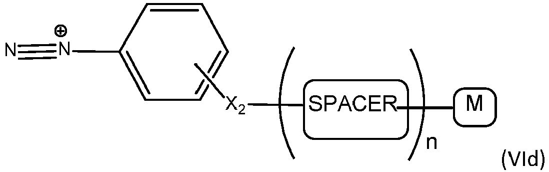

Wherein X₂, spacer, n and M are as defined in formula (Ic)

In another embodiment, the resulting AAV comprises at least one chemically-modified tyrosine of formula (Id). Accordingly, the chemical reagent is of formula (VIe): 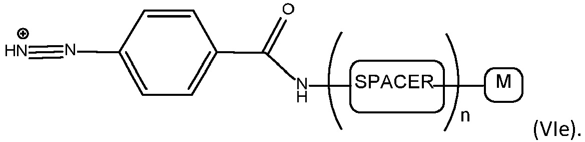 Wherein spacer, n and M are as defined in formula (Id)

The method of the invention may comprise one or several additional steps prior to, or after the step of incubation as described above.

For instance the method of the invention may comprise a step of providing or producing the AAV particles to be chemically modified.

The invention may also comprise a step of providing or preparing the chemical reagent.

The chemical reagent can be produced by synthetic routes. For instance, a chemical reagent of formula (VIIa) may be prepared from the nitro derivative by reducing -NO₂ into -NH₂, e.g. by hydrogenation with Pd/C as catalyst and then forming the aryldiazonium group from the aniline derivative, e.g. by reaction with tBuONO. As illustration only, one can refer to the synthesis of compound of formula II described in the Example section.

The method of the invention may also comprise one or several additional steps following the step of incubation, such as:
- a step of quenching the unreacted chemical reagent at the end of the incubation step and/or
- a step of removing the unreacted reagent, e.g. by dialysis or tangential flow filtration and/or
- a step of collecting the chemically modified AAV particles and/or
- a step of purifying the chemically modified AAV particles and/or
- a step of recovering the chemically modified AAV particles and/or
- a step of formulating and/or packaging the chemically-modified AAV.

The method of the invention may further comprise a step of chemically modifying an amino acid residue other than tyrosine residue of the capsid of the AAV. For instance, said additional chemically-modified amino acid residue may bear an amino group in its side chain.

In a particular embodiment, the method of the invention may comprise a step of incubating the AAV with a chemical reagent of formula (VII): 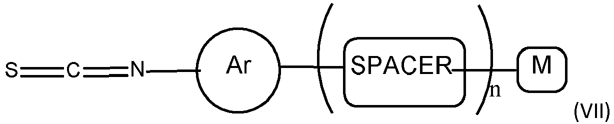 in conditions conducive to promote for reacting said chemical reagent with the amino group of an amino acid residue, e.g. a lysine residue or an arginine residue, present in the capsid of the AAV so as to form a covalent bound. Such a step enables to chemically modify an amino group present in an amino acid residue of the capsid according to the following formula:

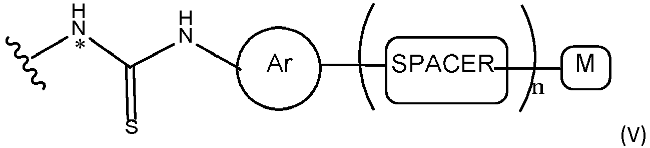

Wherein :
- N* being the nitrogen of the amino group of an amino acid residue, e.g of a lysine residue,
- Ar, Spacer, n and M being as defined in Formula (Ia), (Ib), (Ic) and (Id). It goes without saying that Ar, Spacer, n and M in formula (V) can be the same or different as those present in the at least one chemically-modified tyrosine as described above.

Typically such a step may be performed in an aqueous buffer, such as TRIS buffer, at a pH from 8 to 10, e.g at a pH of about 9.3 and a temperature from 10°C to 50°C, e.g. at room temperature. More details concerning the implementation of such a step can be found in WO2017/212019.

This step can be performed prior, concomitantly or after the step of chemically-modifying at least one tyrosine residue in the capsid of the AAV, as described above.

The invention also relates to the chemically-modified AAV obtainable, or obtained, by the method of the invention as described above.

In a further aspect, the invention also relates to a method for modifying one or several biological properties of the AAV, more precisely that of a recombinant AAV intended to be used as gene vector in gene therapy. Indeed, depending on the nature of "M" moiety, the method for chemically-modifying the capsid of an AAV, more precisely for chemically modifying at least one tyrosine residue in the capsid of an AAV, as described above may enable to:
- Modify the tropism e.g. increase selectivity of the AAV towards a specific organ, tissue or cell (either administered in vivo or transducing tissues or cells in culture) or shift the selectivity of the AAV from one tissue/organ/cell to another, and/or
- Alter immunoreactivity of the AAV, e.g. a decrease immunogenicity of the AAV and/or decrease affinity for neutralizing antibodies, and/or said AAV triggers an altered humoral response when administered in vivo, e.g. do not generate AAV-directed neutralizing antibodies, and/or
- Increase infectivity efficiency of the AAV particles, and/or
- Reduce off-target effects i.e. transduce cells that are not necessary for providing a benefit of the drug and could be even detrimental.
- Increase transduction efficacy of the AAV towards a specific cell, tissue or organ.
- Reduce cellular toxicity when transducing cells in culture
- Induce cellular targeted mortality into cancer cells
- Enabling visualization/monitoring of the AAV particle upon in vivo administration or upon modification of cells in vitro with these AAV particles
- combine a therapeutic agent and a diagnostic agent in the AAV

### • Uses of the AAV of the invention

The chemically-modified AAV of the invention can be used as a research tool or as a medicament, for instance as vectors for the delivery of therapeutic nucleic acids such as DNA or RNA and or as a diagnostic mean e.g. as an imaging agent or combination of both, including theragnostic use.

In some embodiments, the chemically-modified AAV of the invention is used for delivering a nucleic acid into a cell, and is thus a recombinant AAV.

The recombinant AAV can be administered to the cell in vivo, ex vivo or in vitro. The cell may be derived from any mammal including humans, primates, cows, mice, sheeps, goats, pigs, rats, and the like. The cell may be of any type, including hepatocytes, cardiomyocytes, myocytes, neurons, motor neurons, retinal pigmented cells, photoreceptors, chondrocytes, hematopoietic stem cells (HSC) or induced pluripotent stem cells (iPS).

The recombinant AAV of the invention may be used to deliver a therapeutic nucleic acid of interest in a subject. The invention thus relates to a method for delivering a therapeutic nucleic acid of interest in a subject in need thereof comprising administering the chemically-modified AAV of the invention to a subject in need thereof. The recombinant AAV of the invention can be delivered by any appropriate route to the subject. Appropriate administration routes encompass, without being limited to, inhalational, topical, intra-tissue (e.g. intramuscular, intracardiac, intrahepatic, intrarenal), conjunctical (e.g. intraretinal, subretinal), mucosal (e.g. buccal, nasal), intra-articular, intravitreal, intracranial, intravascular (e.g. intravenous), intraventricular, intracisternal, intraperitoneal and intralymphatic routes. Typically, the route of administration is selected depending on the targeted tissue/organ, namely depending on the tissue/organ in which the transduction is sought.

The dose of AAV to administer to the subject is typically determined by the skilled artisan in view of the specific features of the subject, the therapeutic effect sought and the targeted tissue/organ. One single administration or several administrations of the AAV may be requested to achieve the sought therapeutic effect. The AAV of the invention is typically administered in the form of a pharmaceutical composition, namely as a mixture with one or several pharmaceutical excipients.

The conditions to be treated by the administration of the AAV may be of any type, and includes genetic disorders as well as acquired disorders. Genetic disorders of interest encompass genetic muscle disorders such as Duchenne Muscular Dystrophy, leukodystrophy, spinal muscular atrophy (SMA), hemophilia, sickle disease, and inherited retinal dystrophy. The chemically-modified AAV may also be used for treating disorders such as cancers, arthritis, arthrosis, congenital and acquired cardiac diseases, Parkinson disease, Alzheimer's disease as well as infectious diseases such as hepatitis C.

Another object of the invention is a pharmaceutical composition comprising a chemically-AAV of the invention and at least one pharmaceutically acceptable excipients. The pharmaceutical excipients may be selected from well-known excipients such as carriers, preservatives, antioxidants, surfactants, buffer, stabilizer agents, and the like.

The invention further relates to an *in vivo* or *ex vivo* method for delivering a nucleic acid of interest in a cell comprising contacting the chemically-modified AAV of the invention with the cell. The cell may be from the patient. After the transduction, the cell may be transplanted to the patient in need thereof. The cell may be, for instance, hematopoietic stem cells. The nucleic acid of interest may be of any type and is selected depending on the sought effect.

For instance, the AAV may comprise an exogenous gene expression cassette. Said cassette may comprise a promoter, the gene of interest and a terminator. As another example, the AAV of the invention may comprise a DNA template for homologous recombination in cells. Such a recombinant AAV can be used in combination with gene editing tools, for promoting homologous recombination in targeted cells. The gene editing tools can be of any type, and encompass, without being limited to, CRISPR/Cas9, Zinc Finger Nuclease, meganuclease as well as RNA and DNA encoding said proteins.

The invention also relates to a host cell transfected with a chemically modified AAV of the invention, said host cell can be of any type.

For instance, said host cell may be hepatocytes, cardiomyocytes, myocytes, neurons, motor neurons, retinal pigmented cells, photoreceptors, chondrocytes, hematopoietic stem cells (HSC) or induced pluripotent stem cells (iPS).

Further aspects and advantages of the present invention are disclosed in the following experimental section, which should be regarded as illustrative and not limiting the scope of the present application.

### Examples

The following compounds were prepared:

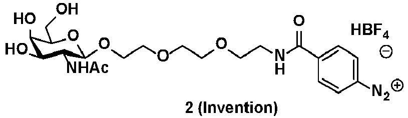

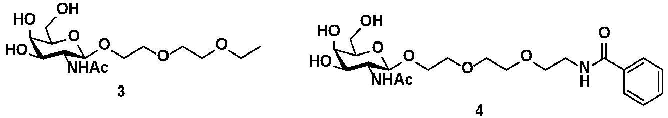

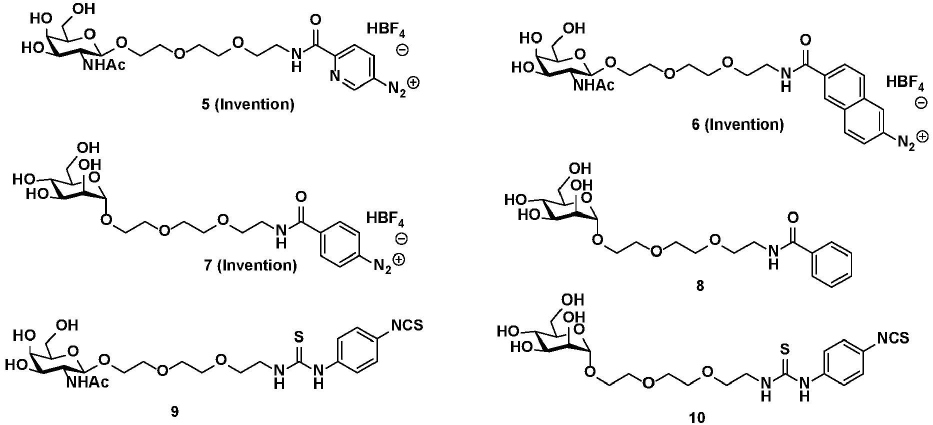

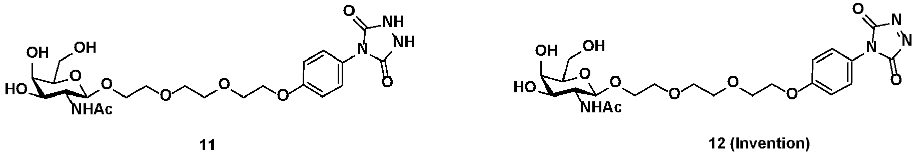

The synthesis of the compounds **3, 9** and **10** and the procedure of coupling on the amino group of the capsid of AAV are already published in Chemical Science, 2020, 11, 1122-1131. The synthesis of other compounds are described hereabove.

### 1. Synthesis of compound 2 (Invention)

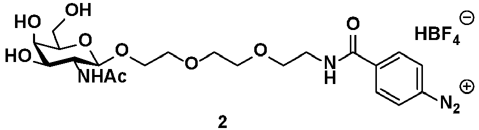

The ligand 2 is prepared as follows:

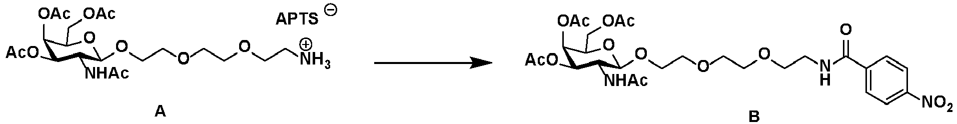

Compound **A** (1 eq, 1.12 mmol) was solubilized in DCM, DMAP (5 eq, 5.6 mmol), 4-nitrobenzoylchloride (3 eq, 3.3 mmol) were added. The mixture was stirred at room temperature for 4 hours. The organic solution was washed with HCl 1M, water and NaHCO₃, dried over MgSO₄. After concentration, the orange oil was purified by by flash chromatography (SiO₂, AcOEt/ MeOH 85/15) . A slightly yellow solid of compound **B** is obtained. Yield: 51.7 %.

NMR ¹H (300 MHz, MeOD): δ (ppm) 8.34 (d, J= 8.98 Hz, 2H), 8.06 (d, J= 9 Hz, 2H), 5.32 (d, J= 3.13 Hz, 1H), 5.06 (dd, J= 3.3 Hz, J= 11 HZ, 1H), 4.61 (d, J= 8.41 Hz, 1H), 4.12 (m, 3H), 3.95 (m, 2H), 3.65 (m, 12H), 2.10 (s, 3H), 2.02 (s, 3H), 1.94 (s, 3H), 1.92 (s, 3H). NMR ¹³C (75 MHz, MeOD): δ (ppm) 173.66, 172.08, 172.05, 171.75, 168.22, 151.04, 141.44, 129.85 x 2C, 124.66 × 2C, 102.87, 72.18, 71.84, 71.60, 71.39, 71.35, 70.39, 70.21, 68.18, 62.73, 51.56, 41.17, 22.92, 20.55 × 2C, 20.51. HRMS: TOF (ESI+) m/z calcd for C₂₇H₃₇N₃O₁₄Na (M+Na)⁺ 650.2173, found 650.2172.

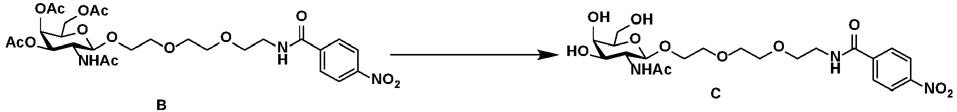

Compound **B** (1 eq, 0.6 mmol) was solubilized in a mixture 1:1 H₂O/MeOH and Amberlist basic resin was added. The mixture was stirred 2 hours until disappearance of the starting material. The resin was filtered and the filtrate concentrated under vacuum to give the corresponding product **C** as a white solid. Yield: Quantitative.

NMR ¹H (400 MHz, MeOD) : δ (ppm) 8.33 (d, J= 8.8 Hz, 2H), 8.05 (d, J= 9.2 Hz, 2H), 4.42 (d, J= 8.4 Hz, 1H), 3.93 (m, 2H), 3.82-3.46 (m, 18H), 1.96 (s, 3H) NMR ¹³C (100 MHz, MeOD) : δ (ppm) 174.12, 168.29, 151.08, 141.44, 129.78, 124.66, 103.12, 76.79, 73.50, 71.62, 71.54, 71.40, 70.41, 69.76, 69. 67, 62.57, 54.29, 41.19, 23.08. HRMS: TOF (ESI+) m/z calcd for C₂₁H₃₁N₃O₁₁Na (M+Na)⁺ 524.1856, found 524.1852.

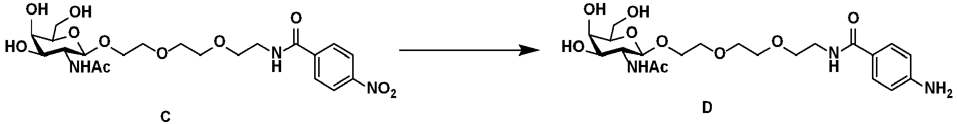

Compound **C** (1 eq, 0.6 mmol) was solubilized in MeOH (15 mL), Pd on charcoal (10% w/w) was added. The mixture was put under H₂ atmosphere and stirred at room temperature for 5 hours. The mixture was filtered on celite and the filtrate concentrated under vacuum to give a white solid corresponding to compound **D.** Yield: 82.3 %.

NMR ¹H (400 MHz, MeOD): δ (ppm) 7.60 (d, J= 8.8 Hz, 2H), 6.67 (d, J= 8.8 Hz, 2H), 4.41 (d, J= 8.4 Hz, 1H), 3.76-3.46 (m, 20H), 1.96 (s, 3H) NMR ¹³C (100 MHz, MeOD): δ (ppm) 174.16, 170.47, 153.15, 129.97, 123.20, 114.76, 103.12, 76.72, 73.49, 71.57, 71.34, 70.79, 69.74, 69.70, 62.56, 54.29, 40.68, 23.09. HRMS: TOF (ESI+) m/z calcd for C₂₁H₃₃N₃O₉Na (M+Na)⁺ 494.2114, found 494.2108.

Diazonium salt derivative **2** was obtained by adding HBF₄ (1.1 eq) and tBuONO (1.1 eq) in ultra-pure water to the amino derivative **D.** Five minutes after voterxing the formation of the diazonium salt was confirmed by using a solution of 2-hydroxynaphtol. The mixture of a drop of each solution give a red coloration allowing the formation of the diazonium salt.

### 2. Synthesis of Compound 4 (Comparative)

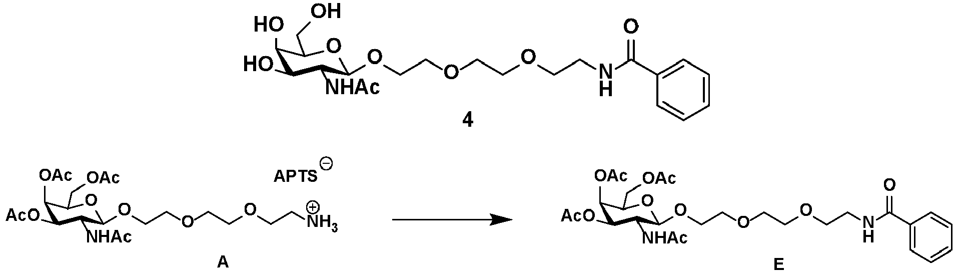

Compound **A** (1 eq, 0.2 mmol) was solubilized in DCM (15 mL), DMAP (5 eq, 1.1 mmol), benzoyl chloride (3 eq, 0.8 mmol) were added. The mixture was stirred at room temperature for 4 hours. The organic solution was washed with HCl 1M, water and NaHCO₃, dried over MgSO₄. After concentration, the yellow oil was purified by flash chromatography (SiO₂, AcOEt/MeOH 8/2). A white solid of compound **E** is obtained. Yield: 67 %.

NMR ¹H (400 MHz, MeOD): δ (ppm) 7.83 (d, J= 8.4 Hz, 2H), 7.48 (m, 3H), 5.31 ( d, J= 3.2 Hz, 1H), 5.06 (dd, J= 11.2 Hz, J= 3.2 Hz, 1H), 4.61 (d, J= 8.4 Hz, 1H), 4.11 (m, 3H), 3.92 (m, 2H), 3.68-3.59 (m, 12H), 2.12( s, 3H), 2.02 (s, 2H), 1.94 (s, 3H), 1.91 (s, 3H). NMR ¹³C (100 MHz, MeOD): δ (ppm) 173.59, 172.07, 172.06, 171.76, 170.31, 135.72, 132.66, 129.56, 128.34, 102.77, 72.23, 71.84, 71.65, 71.46, 71.36, 70.58, 70.10, 68.23, 62.75, 51.65, 40.88, 22.90, 20.56, 20.54, 20.52. HRMS: TOF (ESI+) m/z calcd for C₂₇H₃₉N₂O₁₂ (M+H)⁺ 583.2503, found 583.2504.

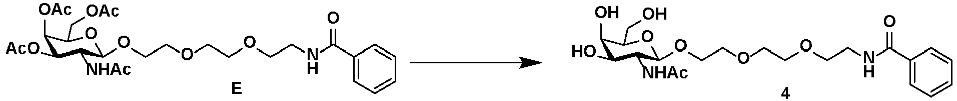

Compound **E** (1 eq, 0.15 mmol) was solubilized in a mixture 1:1 H2O/MeOH and Amberlist basic resin was added. The mixture was stirred for 2 hours until disappearance of the starting material. The resin was filtered and the filtrate concentrated under vacuum to give the corresponding product as a colourless oil. The oil was then freeze-dried to give a white solid of compound **4.** Yield: Quantitative

NMR ¹H (400 MHz, MeOD): δ (ppm) 7.83 (m, 2H), 7,49 (m, 3H), 4.42 (d, J= 8.4 Hz, 1H), 3.93 (m, 2H), 3.81-3.56 (m, 16H), 3.46 (m, 1H), 1.96 (s, 3H) NMR ¹³C (100 MHz, MeOD): δ (ppm) 174.14, 170.37, 135.69, 132.66, 129.57, 128.32, 103.09, 76.77, 73.52, 71.63, 71.58, 71.40, 70.60, 69.73, 69.70, 62.55, 54.32, 40.91, 23.07 HRMS: TOF (ESI+) m/z calcd for C₂₁H₃₂N₂O₉Na (M+Na)⁺ 479.2006, found 479.1997.

### 3. Synthesis of compound 5 (Invention)

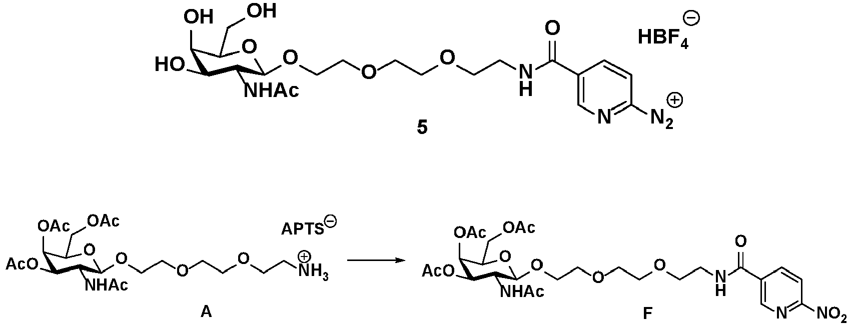

To a solution of A (160 mg, 0.246 mmol) in dry DCM (5 mL) were added 6-nitro-2-nicotinoyl chloride (91 mg, 0.492 mmol) and DMAP (90 mL, 0.738 mmol). After 2 h of stirring at 20°C, the mixture of the reaction was diluted with DCM and washed with 1M HCl and an aqueous saturated solution of NaHCO₃. The combined organic phases were washed with brine, dried with MgSO₄ and the mixture was filtered and concentrated under reduced pressure. The residue was purified by flash chromatography (SiO₂, AcOEt/MeOH: 90/10) to yield the amide F (94 mg, 0.149 mmol) as a white solid. Yield: 61 %.

¹H NMR (300 MHz, CDCl₃): δ = 1.92 (s, 3H, AcO), 1.97 (s, 3H, AcO), 2.01 (s, 3H, AcO), 2.13 (s, 3H, NHAc), 3.63-3.94 (m, 13H), 4.06-4.21 (m, 3H, H-2, H-6a, H-6b), 4.76 (d, 1H, *J*_{1,2} = 8.6 Hz, H-1), 5.12 (dd, 1H, *J*_{3,2} = 11.3 Hz, *J*_{3,4} = 3.5 Hz, H-3), 5.28 (dd, 1H, *J*_{4,3} = 3.4 Hz, *J*_{4,5} = 0.8 Hz, H-4), 6.29 (d, 1H, *J* = 9.1 Hz, NHAc), 8.22 (d, 1H, *J=* 7.9 Hz, Py), 8.37 (d, 1H, *J* = 8.1, *J* = 0.9 Hz, Py), 8.55 (d, 1H, *J=* 7.7, *J=* 0.9 Hz, Py). ¹³C NMR (100.6 MHz, CDCl₃): δ = 20.6 (2 x CH₃, 2 x AcO), 20.7 (CH₃, AcO), 20.69 (CH₃, AcO), 23.2 (CH₃, NHAc), 39.4 (CH₂), 50.9 (CH, C-5), 61.5 (CH₂, C-6), 66.8 (CH, C-4), 68.7 (CH₂), 69.7 (CH₂), 70.49 (CH₂), 70.51 (CH₂) 70.6 (CH), 70.7 (CH, C-3), 71.1 (CH₂), 101.9 (CH, C-1), 120.1 (CH, Py), 127.7 (CH, Py), 141.6 (CH, Py), 149.6 (C, Py), 155.1 (C, Py), 162.2 (C, CO), 170.3 (C, AcO), 170.4 (C, AcO), 170.5 (C, AcO), 170.6 (C, NHAc). HRMS (ESI): m/z calcd for C₂₆H₃₇N₄O₁₄ [M + H]⁺: 629.2306, found: 629.2307

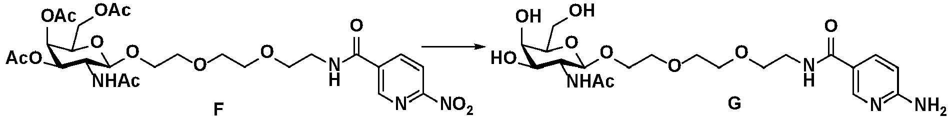

**Compound F** (88 mg, 0.139 mmol) was dissolved in dry MeOH (2.8 mL) and sodium methoxide (42 µL of 1 M solution in MeOH, 0.042 mmol) was added. The mixture was stirred for 2 h, and neutralized by adding acid resin Amberlite IR120 (H), filtered and the solvents evaporated to dryness. The crude of the reaction was used in the next step without further purification.

To a solution of deprotected sugar (0.139 mmol) in MeOH (3 mL) was added Pd/C (24 mg, 20% w). The resulting suspension was stirred under H₂ atmosphere (1 atm) for 8 h. The Pd/C was removed by filtration through Celite^{®} and the filtrate was evaporated under reduced pressure. The crude of the reaction was lyophilized to yield the aniline **G** (54 mg, 0.114 mmol) as a white solid. Yield: 82 %

¹H NMR (300 MHz, CD₃OD): δ = 1.97 (s, 3H, NHAc), 1.49 (t, 2H, *J* = 5.9 Hz), 3.57-3.80 (m, 13H), 3.84 (bd, 1H, *J*= 3.1 Hz), 4.06 (m, 2H), 4.43 (d, 1H, *J*_{1,2} = 8.5 Hz, H-1), 6.69 (dd, 1H, *J* = 8.4 Hz, *J=* 0.8 Hz, Py), 7.30 (dd, 1H, *J* = 7.3, *J* = 0.7 Hz, Py), 7.53 (dd, 1H, *J* = 8.2, *J* = 7.3 Hz, Py). ¹³C NMR (100.6 MHz, CDCl₃): δ = 23.1 (CH₃, NHAc), 40.2 (CH₂), 54.3 (CH, C-5), 62.6 (CH₂, C-6), 69.7 (CH), 69.8 (CH₂), 70.8 (CH₂), 71.40 (CH₂), 71.47 (CH₂), 71.54 (CH₂), 70.6 (CH), 73.4 (CH, C-3), 76.7 (CH), 103.2 (CH, C-1), 112.0 (CH, Py), 113.1 (CH, Py), 139.4 (CH, Py), 148.9 (C, Py), 160.2 (C, Py), 167.5 (C, CO), 174.2 (C, NHAc). HRMS (ESI): m/z calcd for C₂₀H₃₂N₄O₉ [M + Na]⁺: 495.2067, found: 495.2063.

Diazonium salt derivative **5** was obtained by adding HBF₄ (1.1 eq) and tBuONO (1.1 eq) in ultra-pure water to the amino derivative **G.** Five minutes after voterxing the formation of the diazonium salt was confirmed by using a solution of 2-hydroxynaphtol. The mixture of a drop of each solution give a red coloration allowing the formation of the diazonium salt.

### 4. Synthesis of compound 6 (Invention)

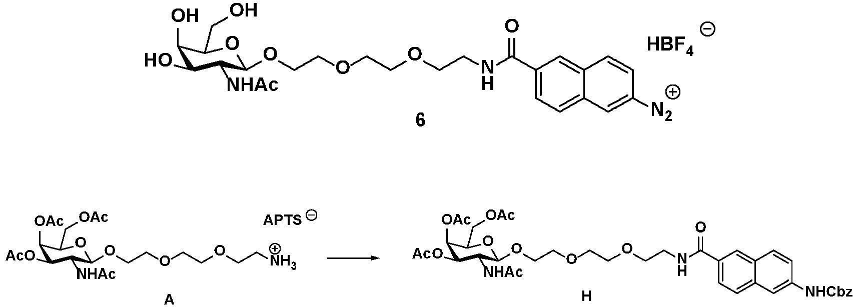

To a solution of 6-(((benzyloxy)carbonyl)amino)-2-naphthoic acid (210 mg, 0.644 mmol) in dry DCM (4.3 mL) and HOBt (87 mg, 0.644 mmol) was added dropwise, at 0°C, DIC (90 mL, 0.738 mmol). After 15min of stirring, a solution of **A** (280 mg, 0.429 mmol) containing TEA (88 µL, 0.644 mmol) was added dropwise. After 2 h of stirring at rt, the mixture of the reaction was diluted with DCM and washed with 1M HCl and an aqueous saturated solution of NaHCO₃. The combined organic phases were washed with brine, dried with MgSO₄ and the mixture was filtered and concentrated under reduced pressure. The residue was purified by flash chromatography (SiO₂, AcOEt/MeOH: 95/5) to yield the amide **H** (94 mg, 0.120 mmol) as a white solid. Yield: 28 %.

¹H NMR (300 MHz, CD₃OD): δ = 1.91 (s, 3H, AcO), 1.94 (s, 3H, AcO), 1.97 (s, 3H, AcO), 2.05 (s, 3H, NHAc), 3.53-3.91 (m, 13H), 4.00 (d, 2H, *J* = 4.0 Hz), 4.07 (dd, 1H, *J* = 11.3 Hz, *J* = 8.5 Hz), 4.42 (s, 2H, PhCH₂), 4.48 (d, 1H, *J*_{1,2} = 8.5 Hz, H-1), 5.00 (dd, 1H, *J*_{3,2} = 11.3 Hz, *J*_{3,4} = 3.4 Hz, H-3), 5.24 (dd, 1H, *J*_{4,3} = 3.4 Hz, *J*_{4,5} = 0.8 Hz, H-4), 6.73 (d, 1H, *J* = 2.3 Hz), 7.06 (d, 1H, *J* = 8.9 Hz, *J* = 2.3 Hz), 7.19-7.41 (m, 5H), 7.53 (d, 1H, *J* = 8.9 Hz), 7.70 (m, 2H), 8.18 (d, 1H, *J* = 1.8 Hz). ¹³C NMR (100.6 MHz, CD₃OD): δ = 20.5 (CH₃, AcO), 20.6 (2 x CH₃, 2 x AcO), 22.9 (CH₃, NHAc), 40.8 (CH₂), 48.3 (CH₂), 51.5 (CH, C-2), 61.7 (CH₂, C-6), 68.1 (CH, C-4), 70.1 (CH₂), 70.7 (CH₂), 71.3 (CH₂), 71.4 (CH₂), 71.6 (CH₂), 71.7 (CH), 72.2 (CH, C-3), 102.8 (CH, C-1), 104.3 (CH, Ar), 120.1 (CH, Ar), 125.2 (CH, Ar), 126.9 (CH, Ar), 127.3 (C, Ar), 127.9 (C, Ar), 128.0 (CH, Ar), 128.4 (2 X CH, Ar), 128.9 (CH, Ar), 129.5 (2 X CH, Ar), 130.9 (CH, Ar), 138.7 (C), 140.8 (C), 149.7 (C), 170.6 (C, AcO), 171.8 (C, AcO), 172.0 (2 x C, AcO, CO), 173.6 (C, NHAc). HRMS (ESI): m/z calcd for C₃₉H₄₈N₃O₁₄ [M + H]⁺: 782.3136, found: 782.3142.

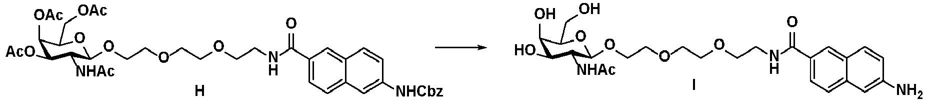

**Compound H** (86 mg, 0.109 mmol) was dissolved in MeOH (2.2 mL) and basic resin (190 mg) was added. The mixture was stirred for 2 h, filtered and the solvents evaporated to dryness. The crude of the reaction was used in the next step without further purification.

To a solution of crude deprotected sugar (0.109 mmol) in MeOH (4 mL) was added Pd/C (17 mg, 20% w). The resulting suspension was stirred under H₂ atmosphere (1 atm) for 6 h. The Pd/C was removed by filtration through Celite^{®} and the filtrate was evaporated under reduced pressure. The crude of the reaction was lyophilized to yield the aniline **I** (51 mg, 0.098 mmol) as a white solid. Yield: 90 %.

¹H NMR (300 MHz, CD₃OD): δ = 1.95 (s, 3H, NHAc), 3.43 (t, 2H, *J* = 5.4 Hz), 3.48-3.84 (m, 21H), 3.92 (m, 2H), 4.38 (d, 1H, *J*_{1,2} = 8.5 Hz, H-1), 6.97 (d, 1H, *J* = 2.2 Hz), 7.05 (dd, 1H, *J* = 8.7 Hz, *J* = 2.2 Hz), 7.57 (d, 1H, *J* = 8.7 Hz), 7.73 (m, 2H), 8.19 (d, 1H, *J=* 1.8 Hz). ¹³C NMR (100.6 MHz, CD₃OD): δ = 23.1 (CH₃, NHAc), 40.9 (CH₂), 54.3 (CH, C-2), 62.5 (CH₂, C-6), 69.68 (CH, C-4), 69.74 (CH₂), 71.4 (CH₂), 71.6 (2 X CH₂), 73.5 (CH), 76.7 (CH, C-3), 103.1 (CH, C-1), 108.3 (CH, Ar), 120.4 (CH, Ar), 125.1 (CH, Ar), 127.7 (C, Ar), 128.3 (C, Ar), 128.9 (CH, Ar), 131.2 (CH, Ar), 138.4 (C), 149.2 (C), 170.7 (C, NHAc), 174.1.0 (C, CO). HRMS (ESI): m/z calcd for C₂₅H₃₅N₃O₉ [M + Na]⁺: 544.2271, found: 544.2270.

Diazonium salt derivative 6 was obtained by adding HBF₄ (1.1 eq) and tBuONO (1.1 eq) in ultra-pure water to the amino derivative I. Five minutes after voterxing the formation of the diazonium salt was confirmed by using a solution of 2-hydroxynaphtol. The mixture of a drop of each solution give a red coloration allowing the formation of the diazonium salt.

### 5. Synthesis of compound 7 (Invention)

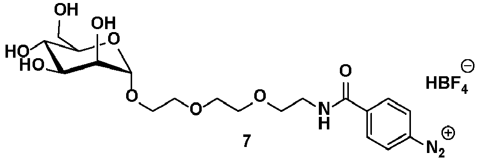

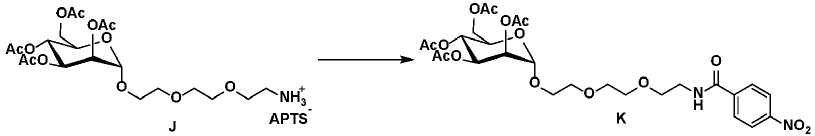

The **compound J** (1 eq, 1.38 mmol, and 0.900 g) was solubilized in DCM, DMAP (3 eq, 4.14 mmol, 0.506 g) 4-nitrobenzoylchloride (3 eq, 4.14 mmol, 0.769 g) was added. The mixture was stirred at RT for 4 hours. The organic solution was washed with HCl 1M, water and NaHCO₃, dried over MgSO₄. After concentration, the orange oil was purified by flash chromatography (SiO₂, AcOEt/MeOH : 85/15) to yield a slightly yellow solid **K.** (0.270 g, 0.43 mmol). Yield: 31.1 %.

¹H NMR (300 MHz, CDCl₃): δ (ppm) 8.27 (m, 2H), 7.99 (m, 2H), 7.19 (br s, 1H), 5.29 (m, 3H, H2, H3, H4), 4.86 (d, 1H, *J* =1.6 Hz, H-1), 4.25 (m, 1H, H-6), 4.07 (m, 2H), 3.87-3.66 (m, 13H, H-5, H-6, PEG), 2.15 (s, 3H, AcO), 2.09 (s, 3H, AcO), 2.03 (s, 3H, AcO), 1.98 (s, 3H, AcO). ¹³C NMR (75 MHz, CDCl₃): δ (ppm) 170.86 (C, CO), 170.30 (2 × C, 2 X CO), 169.79 (C, CO), 165.66 (C, CO), 149.70 (C), 140.37 (C), 128.50 (2 x C, CH), 123.83 (2 x C, CH), 97.81 (CH, C-1), 70.78 (CH₂), 70.43 (CH₂), 70.14 (CH₂), 69.81 (CH), 69.68 (CH), 68.71 (CH₂), 67.48 (CH₂), 66.23 (CH), 62.60 (CH₂), 40.25 (CH₂), 21.00 (CH₃, AcO), 20.88 (CH₃, AcO), 20.82 (2 x CH₃, 2 x AcO). HRMS: TOF (ESI+) m/z calcd for C₂₇H₃₆N₂O₁₅Na 651.2013, found 651.2002.

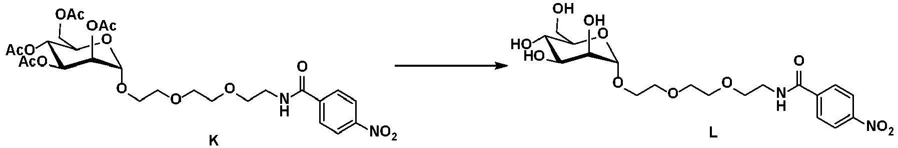

The compound **K** (1 eq, 0.99 mmol, 625 mg) was solubilized in a mixture 1:1 H₂O/MeOH and Amberlist basic resin is added. The mixture was stirred 2 hours until disappearance of the starting material. The resin was filtered and the filtrate concentrated under vacuum to give the product **L** as a white solid. (475 mg, 1 mmol, quant. Not dry).

¹H NMR (300 MHz, MeOD): δ (ppm) 8.32 (m, 2H), 8.04 (m, 2H), 4.72 (d,1H, *J* =1.72 Hz), 3.82 (m, 3H), 3.72-3.54 (m, 16H). ¹³C NMR(75 MHz, MeOD): δ (ppm) 168.28 (C, CO), 151.05 (C), 141.43 (C), 129.75 (2 x CH, Ar), 124.62 (2 x CH, Ar), 101.74 (C-1), 74.63, 72.58, 72.13, 71.61, 71.42, 71.36, 70.47, 68.65, 67.68, 62.98, 41.22 (CH₂). HRMS: TOF (ESI+) m/z calcd for C₁₉H₂₇N₂O₁₁ 459.1615, found 459.1611

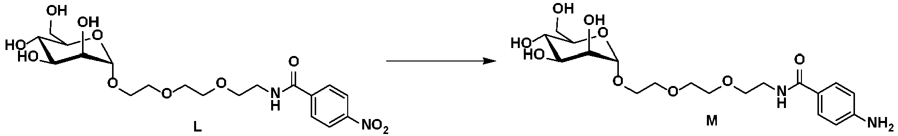

To a solution of **L** (90 mg, 0.197 mmol) in MeOH (3 mL) was added Pd/C (25 mg, 20% w). The resulting suspension was stirred under H₂ atmosphere (1 atm) for 4 h. The Pd/C was removed by filtration through Celite^{®} and the filtrate was evaporated under reduced pressure. The crude of the reaction was lyophilized to yield the aniline **M** (69 mg, 0.160 mmol) as a white solid. Yield: 81 %.

¹H NMR (400 MHz, CD₃OD): δ = 3.52-3.68 (m, 14H), 3.69-3.74 (2H, m), 3.78-3.85 (3H, m), 6.67 (d, 1H, *J* = 8.8 Hz, Ar), 7.71 (d, 1H, *J* = 8.8 Hz, Ar). ¹³C NMR (100.6 MHz, CDCl₃): δ = 40.7 (CH₂), 62.9 (CH₂, C-6), 68.6 (CH), 70.8 (CH₂), 71.28 (CH₂), 71.32 (CH₂), 71.37(CH₂), 71.5 (CH), 72.1 (CH), 72.6 (CH), 74.6 (CH), 101.7 (CH, C-1), 114.8 (2 x CH, Ar), 123.2 (C, Ar), 129.9 (2 x CH, Ar), 153.1 (C, Ar),170.2 (C, amide). HRMS (ESI): m/z calcd for C₁₉H₃₀N₂O₉Na [M + Na]⁺: 453.1849, found: 453.1841.

Diazonium salt derivative 7 was obtained by adding HBF₄ (1.1 eq) and tBuONO (1.1 eq) in ultra-pure water to the amino derivative **M.** Five minutes after voterxing the formation of the diazonium salt was confirmed by using a solution of 2-hydroxynaphtol. The mixture of a drop of each solution give a red coloration allowing the formation of the diazonium salt.

### 6. Synthesis of Compound 8 (Comparative)

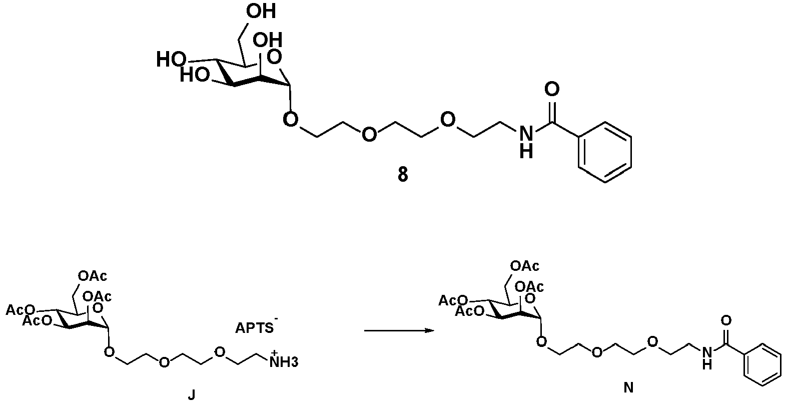

The compound **J** (1 eq, 0.3 mmol, and 0.230 g) was solubilized in DCM, DMAP (3 eq, 0.9 mmol, 0.129 g), 4-nitrobenzoylchloride (3 eq, 0.9 mmol, 0.167 g) is added. The mixture was stirred at RT for 4 hours. The organic solution was washed with HCl 1M, water and NaHCO₃, dried over MgSO₄. After concentration, the orange oil was purified by flash chromatography (SiO₂, AcOEt/MeOH : 85/15) to yield a slightly yellow solid **N.** (70 mg, 0.1 mmol). Yield: 34 %.

¹H NMR (400 MHz, CD₃OD): δ (ppm) 7.82 (m, 2H), 7.49 (m, 3H), 5.26 (m, 3H, H2, H3, H4), 4.87 (d, J=1.36 Hz, 1H), 4.23 (dd, J=5 Hz, J=12,3 Hz, 1H, H6), 4.10 (m, 2H, H5, H6), 3.82 (m, 1H), 3.71-3.58 (m, 12H), 2.12 (s, 3H), 2.05 (s, 3H), 2.02 (s, 3H), 1.95 (s, 3H). ¹³C NMR (100 MHz, CD₃OD): δ (ppm) 172.33 (C, CO), 171.55 (C, CO), 171.49 (C, CO), 171.47 (C, CO), 170.23 (C, CO), 135.69 (C), 132.62 (CH), 129.53 (2 X CH), 128.29 (2 X CH), 98.95 (C-1), 71.59 (CH₂), 71.33 (CH₂), 71.18 (CH₂), 70.74 (CH), 70.73 (CH), 70.63 (CH), 69.78 (C-5), 68.34 (CH₂), 67.30 (CH₂), 63.57 (CH₂, C-6), 40.93 (CH₂), 20.65 (CH₃, AcO), 20.62 (CH₃, AcO), 20.61 (CH₃, AcO), 20.55 (CH₃, AcO). HRMS: TOF (ESI+) m/z calcd for C₂₇H₃₈N0₁₃ 584.2343, found 584.2344

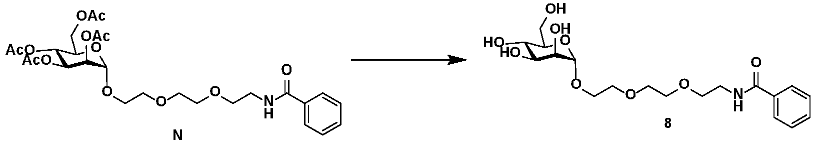

The compound **N** (1 eq, 0.1 mmol, 70 mg) was solubilized in a mixture 1:1 H₂O/MeOH and Amberlist basic resin was added. The mixture was stirred 2 hours until disappearance of the starting material. The resin was filtered and the filtrate concentrated under vacuum to give the corresponding product **8** as a white solid. (50 mg, 0.1 mmol, quant.).

¹H NMR (400 MHz, D₂O): δ (ppm) 7.84 (m, 2H), 7.69 (m, 1H), 7.60 (m, 2H), 4.88 (d, J=1.68 Hz, 1H), 3.97 (m, 1H), 3.93-3.63 (m, 18H). ¹³C NMR (100 MHz, D₂O): δ (ppm) 171.11 (C, CO), 133.65 (C), 132.21 (CH, Ar), 128.83 (2 CH, Ar), 127.10 (2 CH, Ar), 99.93 (C-1), 72.74, 70.54, 69.99, 69.66, 69.58, 68.89, 66.76, 66.37, 60.93, 39.57. HRMS: TOF (ESI+) m/z calcd for C₁₉H₂₉NO₉Na 438.1740, found 438.1730.

### 7. Synthesis of Compound 11 (Comparative)

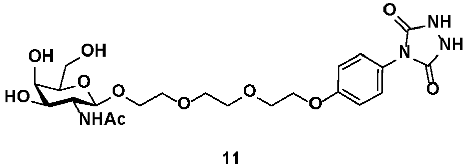

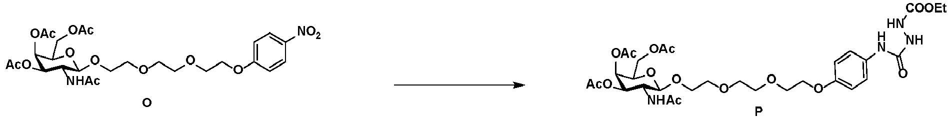

A mixture of compound **O** (550 mg, 0.917mmol) and 20% Pd on carbon (110 mg) in ethyl acetate (9.2 mL) was stirred under a hydrogen atmosphere (1 atm) at room temperature for 2 h. The reaction mixture was filtered through a pad of Celite and the solvent was evaporated in vacuo to give the hydrogenated product as an amorphous yellow solid, used without further purification.

To a solution of CDI (149 mg, 0.917 mmol) in dry THF (9.2 mL) was added ethyl carbazate (95 mg, 0.917 mmol) and stirred at room temperature under nitrogen for 15 min. The crude (0.917 mmol, solved in 4 mL THF) was then added dropwise and the stirring continued overnight. The mixture was diluted with AcOEt, and washed with 0.5 M HCl aqueous solution. The organic phase was dried and concentrated under reduced pressure. The residue was purified by flash chromatography (SiO₂, EtOAc-MeOH, 90:10) to give **P** (311 g, 0.541 mmol) as white solid. Yield: 59%.

¹H NMR (300 MHz, CDCl₃): δ = 1.25 (t, 3H, *J* = 7.1 Hz, CH₃CH₂O), 1.88 (s, 3H, AcO), 1.92 (s, 3H, AcO), 2.03 (s, 3H, AcO), 2.09 (s, 3H, NHAc), 3.59-4.10 (m, 16H), 4.16 (d, 2H, *J*= 7.1 Hz, CH₃CH₂O), 4.73 (d, 1H, *J* = 8.6 Hz, H-1), 5.05 (dd, 1H, *J*_{3,2}= 11.2 Hz, *J*_{3,4} = 3.3 Hz, H-3), 5.26 (bd, 1H, *J*_{4,3} = 3.3 Hz, H-4), 6.78 (d, 3H, *J* = 9.0 Hz, Ph, NH), 7.16 (s, 1H, NH), 7.22 (s, 1H, NH), 7.24 (d, 2H, *J* = 9.0 Hz, Ph), 7.67 (s, 1H, NH). ¹³C NMR (100.6 MHz, CDCl₃): δ = 14.4 (CH₃, CH₃CH₂O), 20.7 (3 x CH₃, 3 x AcO), 23.0 (CH₃, NHAc), 50.4 (CH, C-5), 61.6 (CH₂, C-6), 62.3 (CH₂, CH₃CH₂O), 66.7 (CH, C-4), 67.6 (CH₂), 68.6 (CH₂), 69.9 (CH₂), 70.5 (CH, CH₂), 70.83 (CH), 70.86 (CH₂), 71.2 (CH₂), 101.9 (CH, C-1), 114.9 (2 x CH, Ph), 121.7 (2 x CH, Ph), 131.5 (C, Ph), 154.8 (C), 156.4 (C), 157.5 (C), 170.4 (C, AcO), 170.5 (C, AcO), 170.7 (C, AcO), 171.1 (C, NHAc). HRMS (ESI): m/z calcd for C₃₀H₄₄N₄O₁₅Na [M + Na]⁺: 723.2701, found: 723.2700.

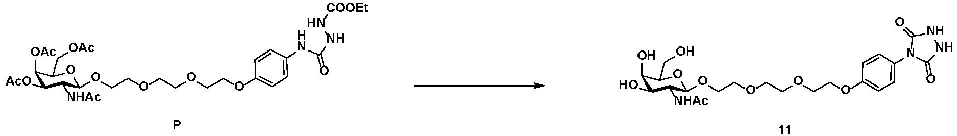

Compound **P** (286 mg, 0.497 mmol) was dissolved in dry MeOH (10 mL) and sodium methoxide (149 µL of 1 M solution in MeOH, 0.149 mmol) was added. The mixture was stirred for 4 h, K₂CO₃ (137 mg, 0.994 mmol) was added and the resulting mixture was stirred at 75 °C for 1.5 h (µW, 50 W). The mixture was diluted with 10 mL H₂O, neutralized with Amberlite IR120 (H), filtered and the solvents evaporated to dryness. The substrate was dissolved in water and subjected to lyophilization give 11 (226 mg, 0.427 mmol) as brown solid. Yied: 86%.

¹H NMR (300 MHz, MeOD): δ = 1.97 (s, 3H, NHAc), 3.45 (ddd, 1H, *J=* 6.5 Hz, *J=* 5.3 Hz, *J* = 1.0 Hz, H-5), 3.53 (dd, 1H, *J*_{3,2} = 10.7 Hz, *J*_{3,4} = 3.3 Hz, H-3), 3.62-3.79 (m, 10H), 3.86-3.97 (m, 4H), 4.19 (m, 2H), 4.44 (d, 1H, *J*_{1,2} = 8.4 Hz, H-1), 6.78 (d, 3H, *J=* 9.0 Hz, Ph, NH), 7.24 (d, 1H, *J=* 9.1 Hz, Ph), 7.35 (d, 1H, *J=* 9.1 Hz, Ph). ¹³C NMR (100.6 MHz, CDCl₃): δ = 23.0 (CH₃, NHAc), 54.4 (CH, C-5), 62.6 (CH₂, C-6), 68.9 (CH₂), 69.7 (CH), 70.8 (CH2), 71.6 (CH2), 71.7 (CH2), 71.8 (CH2), 73.6 (CH), 76.7 (CH), 103.1 (CH, C-1), 116.2 (2 x CH, Ph), 125.6 (C, Ph), 129.0 (2 x CH, Ph), 156.1 (2 X C, Urazol), 160.1 (C, Ph), 174.2 (C, NHAc). HRMS (ESI): m/z calcd for C22H31N4O11 [M - H]+: 527.1989, found: 527.1974.

### 8. Synthesis of compound 12 (Invention)

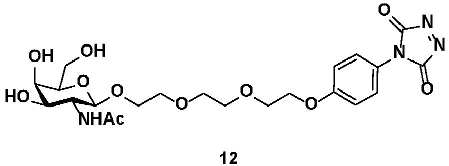

Compound **12** was obtained after oxidation of compound **11** in the presence of 1.1 eq of hydantoin in acetonitrile. A red coloration of the solution confirmed the formation of the PTAD derivative **12.**

### 3. Preparation of chemically-modified AAV

### - AAV2 and AAV8 production and purification

AAV2 vectors were produced from two plasmids: pHelper, PDP2-KANA encoding AAV Rep2-Cap2 and adenovirus helper genes (E2A, VA RNA, and E4) and pVector ss-CAG-LuceGFP or pVector ss-CAG-eGFP. AAV8 vectors were produced from two plasmids: pHelper, PDP8-KANA encoding AAV Rep2-Cap8 and adenovirus helper genes (E2A, VA RNA, and E4) pVector ss-CAG-eGFP. All vectors were produced by transient transfection of HEK293 cells with calcium phosphate-HeBS method. Vector producer cells were harvested 48 hours after transfection. For extraction of AAV2 particles, cells were treated firstly with Triton-1% and benzonase (25U/mL) for 1 hour at 37°C. This step was omitted for AAV8 vectors but subsequent steps were identical. The bulk was centrifuged at 2000 rpm for 20 min and subjected to freeze-thaw cycles to release vector particles. The cellular debris were removed by centrifugation at 2500 rpm for 15 min. Cell lysates were precipitated with PEG overnight and clarified by centrifugation at 4000 rpm for 1 hour. The precipitates were then incubated with benzonase for 30 min at 37 °C and collected after centrifugation at 10000 g for 10 min at 4°C. Vectors were purified by double cesium chloride (CsCl) gradient ultracentrifugation. The viral suspension was then subjected to four successive rounds of dialysis under slight stirring in a Slide-a-Lyzer cassette (Pierce) against dPBS (containing Ca⁺⁺ and Mg⁺⁺).

### - Coupling and purification

Diazonium salts derivatives **2, 5, 6** and **7** were obtained by adding HBF₄ (1.1 eq) and tBuONO (1.1 eq) in ultra-pure water to the amino corresponding compounds. Five minutes after voterxing the formation of the diazonium salt was confirmed by using a solution of 2-hydroxynaphtol. The mixture of a drop of each solution give a red coloration allowing the formation of the diazonium salt. Then, AAV2-GFP-Luc, AAV2-GFP or AAV8-GFP (1E12 vg, 2.49 nmol) were added to a solution of TRIS buffer pH = 9.3 containing **2** (invention), **3** (comparative), **4** (comparative), **5** (invention), **6** (invention), **7** (invention) or **8** (comparative) at a molar ratio of 3E5 or 3E6 equivalents and incubated during 4h at 20°C. The solutions containing the vectors were then dialyzed against dPBS + 0.001% Pluronic to remove free molecules that were non-coupled to the AAV capsid.

For the modification of the amino group of the capsid of AAV2 with compound **9** and **10** the procedure is described in Chemical Science, 2020, 11, 1122-1131.

For the double modification of the tyrosine and the amino group of AAV2-GFP, the coupling was first done with **2** and the procedure of the tyrosine modification and five minute after, with the procedure of the amino group modification using compound **10** at a molar ratio of 3E5 equivalents and incubated during 4h at 20°C. The solutions containing the vectors were then dialyzed against dPBS + 0.001% Pluronic to remove free molecules that were non-coupled to the AAV capsid.

For the PTAD derivative **12** (invention, 3E6 eq) and the urazole derivative **11** (comparative, 3E6 eq), the compounds were added in small portion to the AAV2-GFP in TBS buffer. Five minutes after the addition, the solutions containing the vectors were then dialyzed against dPBS + 0.001% Pluronic to remove free molecules that were non-coupled to the AAV capsid.

### 4. Characterization of chemically-modified AAVs (invention and comparative)

### Material and Methods

### - Viral genome extraction

3µL of AAV2, AAV8 or chemically modified AAV2 or AAV8 were treated with 20 units of DNase I (Roche #04716728001) at 37°C for 45 min to remove residual DNA in vector samples. After the treatment with DNase I, 20µL of proteinase K 20mg/mL (MACHEREY-NAGEL # 740506) was then added and incubated at 70°C for 20 min. Extraction column (NucleoSpin^{®}RNA Virus) were then used to extract DNA from purified AAV vectors.

### - Quantitative real time PCR analysis

Quantitative real time PCR (qPCR) was performed with a StepOnePlus^{™} Real-Time PCR System Upgrade (Life technologies). All PCRs were performed in a 20µL final volume PCR including primers, probe, PCR Master Mix (TaKaRa) and 5µL of template DNA (plasmid standard, or sample DNA). qPCR was carried out with an initial denaturation step at 95°C for 20 seconds, followed by 45 cycles of denaturation at 95°C for 1 second and annealing/extention at 56°C for 20 seconds. Plasmid Standard were generated with seven serial dilutions (containing 10⁸ to 10² copies of plasmid) of a plasmid pTR-UF-11 (ATCC ^{®} MBA-331^{™}) linearized by Sca-I Restriction Enzyme.

### - Western Blotting

All vectors were denatured at 100°C using laemmli sample buffer for 5 min and separated by SDS-PAGE 10% Tris-Glycine polyacrylamide gels (Life Technologies). Precision plus Protein All Blue Standards (BioRad) was used as a molecular-weight size marker. After transferring the proteins to nitrocellulose membrane using a Transfer buffer (25mM tris/192mM Glycine/0.1 (w/v) SDS/20%MeOH) for 1 hour at 150 mA in a Trans-Blot SD Semi-Dry Transfer Cell (Bio-Rad), the membrane was saturated with 5% semi-skimmed milk in PBS-Tween (0.1%) or with 1% gelatin, 0.1% Igepal in PBS-Tween (0.01%) during 2h at RT. After saturation, the membrane was probed with antisera to AAV2 and chemically modified AAV2 (polyclonal or B1 monoclonal),with FITC-Soybean Agglutinin or FITC-Concanavalin A overnight at 4°C. Three washings were carried out between each stage to remove unbound reagents with PBS-Tween (0.1%) for 15 min at RT. Bands were visualized by chemiluminescence using alkaline phosphatase (AP) or horseradish peroxidase (HRP) conjugated secondary antibodies and captured on X-ray film.

### - Immuno dot-blot

Non-denatured AAV2 or AAV8 and chemically modified AAV2 or AAV8 were deposited on a nitrocellulose paper soaked briefly in PBS prior to assembling the dot blot manifold (Bio-Rad). Nitrocellulose membrane was treated as for western blotting. To visualize the assembled capsid, the membrane was probed with antisera to AAV2 capsid (A20 antibody) or antisera to AAV8 (ADK8 antibody).

### - Infectivity by FACS analysis

HEK293 and HUH7 cells were seeded in a 24-well plate at a density of approximately 2.5x105 cells/well in 2mL of DMEM growth medium. Cells were then incubated overnight at 37°C to reach 50% confluence at 37°C and 5% CO2. Cells were transduced at MOI of 1^{E}4 and 1^{E}5 with AAV2 or chemically modified AAV2 vectors in culture medium. All AAV vectors encoded for GFP. The percentage of GFP positive cells was measured by FACS analysis 48h after the transduction. Cells were dissociated with Trypsin-EDTA (Sigma-Aldrich), fixed with 4% paraformaldehyde and analysed on a BD-LSRII Flow Cytometer (BD Bioscience). All data were processed by FlowJo (V10; Flowjo LLC, Ashland, OR).

### - Results

### The results are shown in Figures 1-11

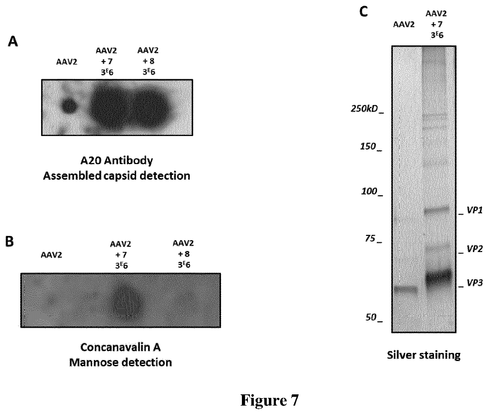

To evaluate the purity and integrity of these GalNAc-AAV2 and GalNAc-AAV8, silver staining of the different samples was carried out. As shown in Fig. 1-A, Fig. 4-C, Fig.6-C and Fig. 8-C and as expected, the ratio VP 1: VP2: VP3 remained intact after undergoing the reaction and subsequent dialysis. To evaluate the purity and integrity of Man-AAV2, silver staining of samples was carried out. As shown in Fig. 7-C and as expected, the ratio VP1:VP2:VP3 remained intact after undergoing the reaction and subsequent dialysis. For GalNAc-AAV2, GalNAc-AAV8 and Man-AAV2 the molecular weight of each VP increased with the coupling of ligand 2, 6 and 7.

The coupling reaction of the sugar at the AAV surface was further studied by dot blot analysis using the soybean lectin and concanavalin A lectin that binds selectively to GalNAc and Mannose residues respectively. Immunostaining with A20 antibody which recognize the assembled AAV2 capsid and the ADK8 antibody that bind to assembled AAV8 capsid were also performed respectively. The positive dots with A20 antibody and with ADK8 antibody indicated that AAV2 and AAV8 remained intact after the coupling procedure with compounds **2, 4, 6, 7, 8, 9 10, 11** and **12** and subsequent dialysis **(**Fig. 2-A, Fig. 5-A, Fig. 6-A, Fig. 7-A,Fig. 8-A and Fig. 9-A).

Positive signals with soybean lectin were only observed when AAV2 was co-incubated with the diazonium compounds **2** and **6** and the PTAD derivative **12** and when AAV8 was co-incubated with compound **2,** revealing the efficient click tyrosine reaction on the AAV2 and AAV8 capsid (Fig. 2-B, Fig. 6-B,Fig. 8-B and Fig. 9-B). Indeed, no detection was observed with compounds **4** lacking the diazonium group and **11** lacking the PTAD group proving that compound **2, 6** and **12** (invention) were covalently linked, and not physically adsorbed, to AAV2 and AAV8 capsid.

Positive signal with concanavalin A lectin which binds to mannose was observed when AAV2 was co-incubated with compound 7 **(invention),** revealing the efficient click tyrosine reaction on the AAV2 (Fig. 7-B). Indeed, no detection was observed with compound **8 (comparative)** lacking the diazonium group, proving that compound 7 was covalently linked, and not physically adsorbed, to AAV2.

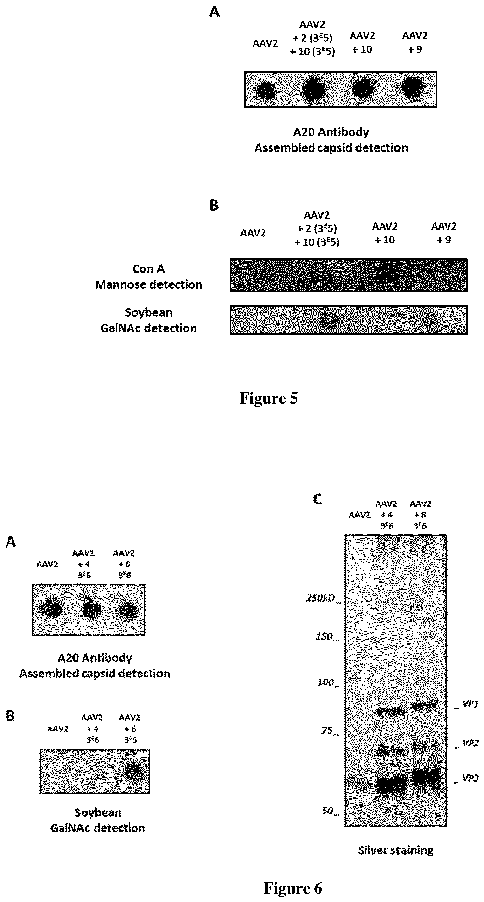

For the double modification of the tyrosine and amino groups of the capsid of AAV2, the co-incubation with **2** and **10** sequentially showed positive dots with concanavalin A and soybean lectins revealing the efficient click tyrosine reaction and amino group modification on the AAV2 (Fig. 5-B).

For Western blot analyses, the use of a capsid specific polyclonal antibody indicated that VP capsid subunits remained intact regardless of the chemical process for AAV2-Luc and AAV2-GFP (Fig. 3-A, Fig. 4-A). Importantly, the band detected with the lectin clearly showed that the compound 2 (invention) is covalently linked to the VPs for AAV2-Luc and AAV2-GFP (Fig. 3-B,C, Fig. 4-B).

On the contrary, the capsid subunits from AAV2-Luc and AAV2-GFP incubated with 3 and **4 (comparative)** at the same ratio did not yield positive bands after incubation with the specific lectin indicating that no coupling occurred without the diazonium function (Figure 3-B,C, Fig. 4-B).

The impact of chemical coupling with compound **2** (invention), which bears GalNAC moiety, on the infectivity of AAV2-GFP vectors was assessed.

The infectivity was evaluated by FACS analysis on HEK293 and HuH7 cells. Both the % of cells expressing GFP as well as the intensity of GFP was measured. As showed in **Fig. 10,** and **Fig. 11** the GalNAC-AAV2-GFP particles are infectious on the two cells lines. The chemical modification of these particles have an important impact on the transduction efficiencies and de-targeting properties. When using a hepatic cell line HuH7, the viral particles chemically modified with compound 2 showed a higher level of transduction than that obtained when using the non-hepatic cell line HEK293.

## Claims

1. An adeno-associated Virus (AAV) having at least one chemically-modified tyrosine residue in its capsid, wherein said chemically-modified tyrosine residue is of formula (I): Wherein: 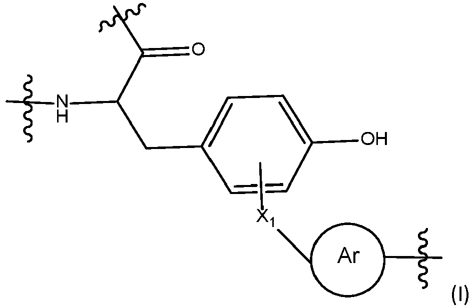
- X₁ is selected from the group consisting of: 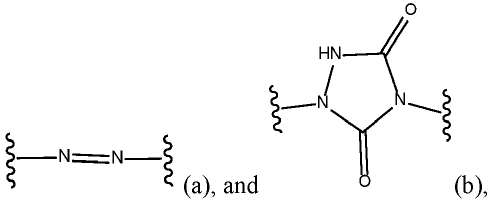 and
- Ar is an aryl or a heteroaryl moiety optionally substituted.

2. The AAV of claim 1, wherein the at least one chemically-modified tyrosine residue is of formula (Ia): 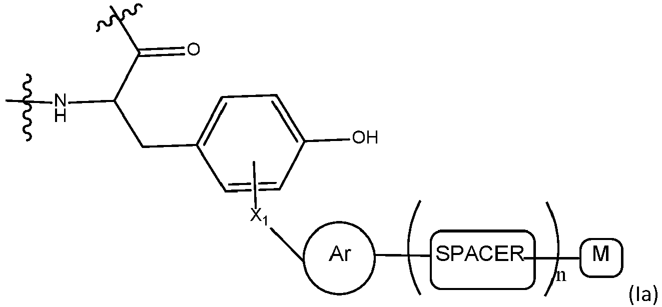 Wherein
- X₁, and Ar are as defined in claim 1,
- Spacer is a group for linking the "Ar" group to the functional moiety "M" which preferably comprises up to 1000 carbon atoms and which is preferably in the form of a chemical chain which optionally comprises heteroatoms and/or cyclic moieties, ,
- n is 0 or 1, and
- M is a functional moiety comprising a steric shielding agent, a labelling agent, cell-type specific ligand or a drug moiety.

3. The AAV of claim 2 wherein X₁ is 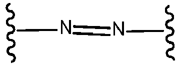 and/or "Ar" is selected from substituted or unsubstituted phenyl, pyridyl, naphthyl, and anthracenyl.

4. The AAV of any one of claims 1 to 3, wherein the at least one chemically-modified tyrosine is of formula (1c): 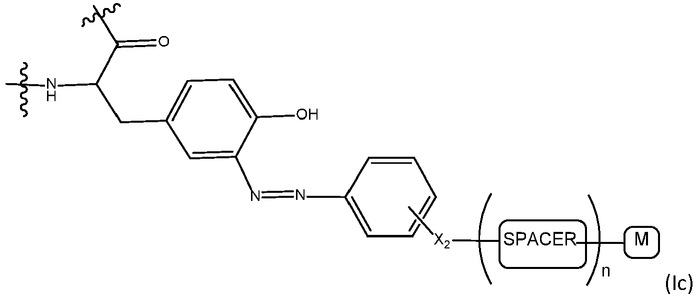 wherein:
- X₂ is -C(=O)-NH, -C(=O)-O, -C(=O)-O-C(=O)-, O-(C=O)-, NH-C(=O)-, NH-C(=O)-NH, -O-C=O-O-, O, NH, -NH(C=S)-, or -(C=S)-NH-, preferably -(C=O)-NH- or -(C=O)-O-
- X₂ is at position para, meta or ortho, preferably at position para of the phenyl group,
- Spacer, n and M are as defined in claim 2.

5. The AAV of any one claims 2 to 4, wherein:
- "Spacer", when present, is selected from the group consisting of saturated or unsaturated, linear or branched C₂-C₄₀ hydrocarbon chains, optionally substituted, polyethylene glycol, polypropylene glycol, pHPMA, PLGA, polymers of alkyl diamines and combinations thereof, and/or
- "M" " comprises, or consists of, cell-type targeting ligand, preferably selected from a mono- or a polysaccharide, a hormone, including a steroid hormone, a peptide such as RGD peptide, a muscle targeting peptide (MTP) or Angiopep-2, a protein or a fragment thereof, a membrane receptor or a fragment thereof, an aptamer, an antibody including heavy-chain antibody, and fragments thereof such as Fab, Fab', and VHH, a ScFv, a spiegelmer, a peptide aptamer, vitamins and drugs such as CB1 and/or CB2 ligands.

6. The AAV of any one claims 2 to 4, wherein:
- "Spacer" (when present) is selected from the group consisting of linear or branched C₂-C₂₀ alkyl chains, polyethylene glycol, polypropylene glycol, pHPMA, PLGA, polymer of alkyl diamine and combinations thereof, said polymers having from 2 to 20 monomers and/or
- "M" comprises, or consists of, a cell-type specific ligand derived from a protein selected from transferrin, Epidermal Growth Factor (EGF), and basic Fibroblast Growth Factor βFGF, a mono- or a polysaccharide comprising one or several galactose, mannose, N-acetylgalactosamine residues, bridge GalNac, or mannose-6-phosphate, MTP selected from SEQ ID NO: 1 to SEQ ID NO:7, and vitamins such as folic acid.

7. The AAV of any one of claims 2 to 6, wherein:
- M is a cell-type specific ligand for specifically targeting hepatocytes and comprises at least one moiety of formula (III): 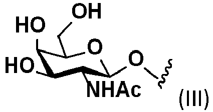 and/or
- Spacer is a polyethylene glycol chain comprising from 2 to 10 monomers.

8. The AAV of claim 7 wherein the at least one chemically modified tyrosine is of formula (II):

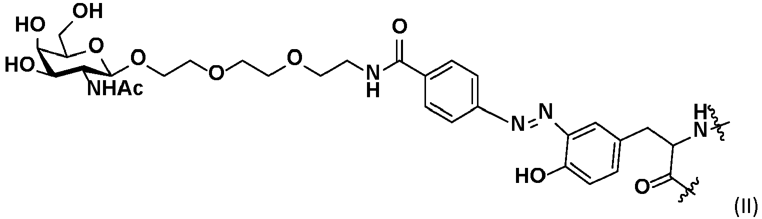

9. The AAV of any one of claims 1 to 8, which further has at least one additional chemically modified amino acid residue in the capsid, which is different from a tyrosine residue, said amino acid residue preferably bearing an amino group chemically modified with a group of formula (V): 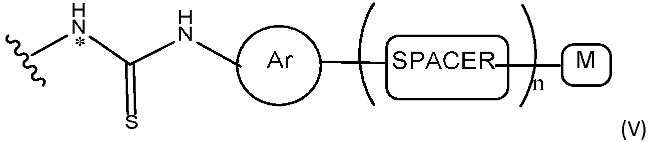 Wherein :
- N* being the nitrogen of the amino group of an amino acid residue, e.g of a lysine residue or arginine residue,
- Ar, Spacer, n and M has the same definition as Ar, Spacer, n and M of formula (II) of claim 2.

10. The AAV of any one of claims 1-9, wherein the AAV is a recombinant AAV, preferably selected from AAV having a wildtype capsid, naturally-occurring serotype AAV, variant AAV, pseudotype AAV, AAV with hybrid or mutated capsids, and self-complementary AAV.

11. A method for chemically-modifying the capsid of an AAV, more precisely for chemically modifying at least one tyrosine residue in the capsid of an AAV, which comprises incubating said AAV with a chemical reagent bearing a reactive group selected from an aryl diazonium, and a 4-phenyl-1,2,4-triazole-3,5-dione (PTAD) moiety in conditions conducive for reacting said reactive group with a tyrosine residue present in the capsid of the AAV so as to form a covalent bound.

12. The method of claim 11, which comprises incubating the AAV with a chemical reagent of formula (VId) 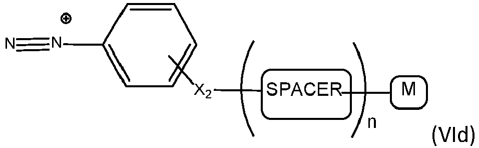 so as to obtain at least one chemically-modified tyrosine residue in the capsid of formula (Ic): 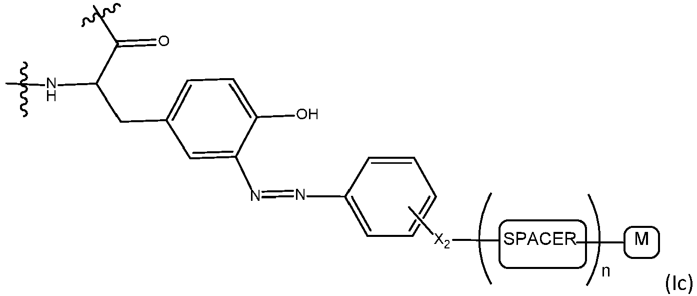 Wherein:
- X₂ is -C(=O)-NH, -C(=O)-O, -C(=O)-O-C(=O)-, O-(C=O)-, NH-C(=O)-, NH-C(=O)-NH, -O-C=O-O-, O, NH, -C=S-NH, or NH-C=S- preferably -(C=O)-NH- or -(C=O)-O-
- X₂ is at position para, meta or ortho, preferably at position para of the phenyl group,
- Spacer is a hydrocarbon group comprising from 2 to 400 carbon atoms,
- n is 0 or 1, and
- M is a chemical moiety comprising a steric shielding agent, a labelling agent, cell-type specific ligand or a drug moiety.

13. A pharmaceutical composition comprising an AAV as defined in any one of claims 1-10 and at least one pharmaceutically acceptable excipient.

14. An AAV of claims 1-10, or a pharmaceutical composition of claim 13, for use as a diagnostic agent or as a drug *in vivo,* preferably in gene therapy.

15. Use of an AAV of claims 1-10, or a pharmaceutical composition of claim 13 as a diagnostic agent and as a gene vector *ex vivo* or *in vitro.*

## Patentansprüche

1. Adeno-assoziiertes Virus (AAV) mit mindestens einem chemisch modifizierten Tyrosinrest in seinem Kapsid, wobei dieser chemisch modifizierte Tyrosinrest die Formel (I) hat: 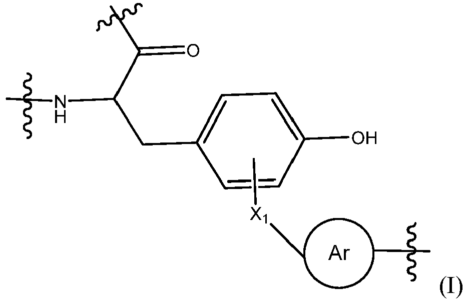 wobei:
- X₁ ausgewählt ist aus der Gruppe bestehend aus: 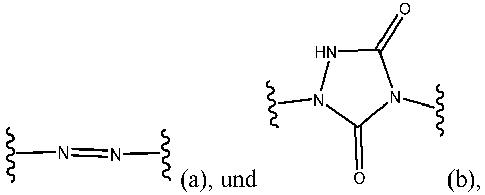 und
- Ar eine Aryl- oder eine Heteroaryleinheit ist, die gegebenenfalls substituiert ist.

2. AAV nach Anspruch 1, wobei der mindestens eine chemisch modifizierte Tyrosinrest die Formel (Ia) hat: 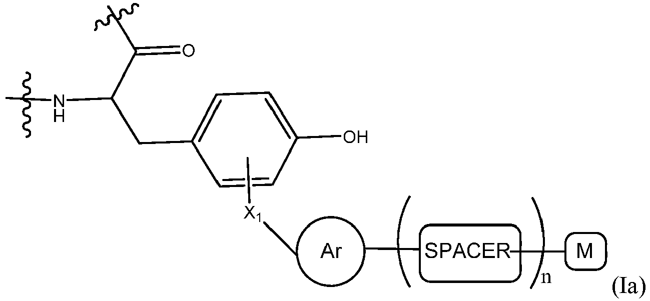 wobei
- X₁, und Ar wie in Anspruch 1 definiert sind,
- Spacer eine Gruppe zur Verknüpfung der "Ar"-Gruppe mit der funktionellen Einheit "M" ist, die vorzugsweise bis zu 1000 Kohlenstoffatome umfasst und die vorzugsweise in Form einer chemischen Kette vorliegt, die gegebenenfalls Heteroatome und/oder zyklische Einheiten umfasst
- n 0 or 1 ist, und
- M eine funktionelle Einheit ist, die ein sterisches Abschirmungsmittel, ein Markierungsmittel, einen zelltypspezifischen Liganden oder eine Arzneimitteleinheit umfasst.

3. AAV nach Anspruch 2 wobei X₁ 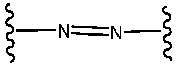 ist und/oder "Ar" ausgewählt ist aus substituiertem oder unsubstituiertem Phenyl, Pyridyl, Naphthyl und Anthracenyl.

4. AAV nach einem der Ansprüche 1 bis 3, wobei das mindestens eine chemisch modifizierte Tyrosin die Formel (1c) hat: 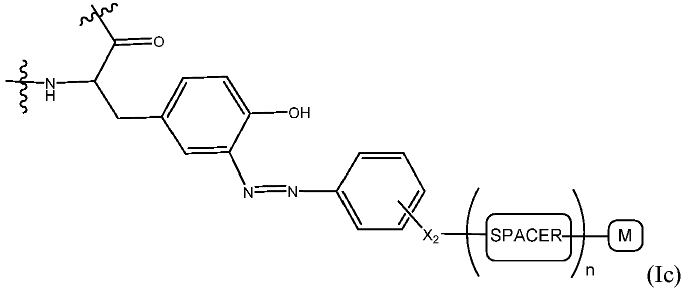 wobei:
- X₂ -C(=O)-NH, -C(=O)-O, -C(=O)-O-C(=O)-, O-(C=O)-, NH-C(=O)-, NH-C(=O)-NH, -O-C=O-O-, O, NH, -NH(C=S)- oder -(C=S)-NH- ist, vorzugsweise -(C=O)-NH- oder -(C=O)-O-,
- X₂ in para-, meta- oder ortho-Stellung, vorzugsweise in para-Stellung der Phenylgruppe steht,
- Spacer, n und M wie in Anspruch 2 definiert sind.

5. AAV nach einem der Ansprüche 2 bis 4, wobei:
- "Spacer", sofern vorhanden, ausgewählt ist aus der Gruppe bestehend aus gesättigten oder ungesättigten, linearen oder verzweigten C₂-C₄₀-Kohlenwasserstoffketten, optional substituiert, Polyethylenglykol, Polypropylenglykol, pHPMA, PLGA, Polymere von Alkyldiaminen und Kombinationen davon, und /oder
- "M" " umfasst oder besteht aus einem Zelltyp-Targeting-Liganden, vorzugsweise ausgewählt aus einem Mono- oder einem Polysaccharid, einem Hormon, einschließlich eines Steroidhormons, einem Peptid wie RGD-Peptid, einem Muskel-Targeting-Peptid (MTP) oder Angiopep-2, einem Protein oder einem Fragment davon, einem Membranrezeptor oder einem Fragment davon, einem Aptamer, einem Antikörper, einschließlich Schwerketten-Antikörper ("heavy-chain antibody"), und Fragmenten davon, wie Fab, Fab' und VHH, einem ScFv, einem Spiegelmer, einem Peptidaptamer, Vitaminen und Arzneimitteln wie CB1- und/oder CB2-Liganden.

6. AAV nach einem der Ansprüche 2 bis 4, wobei:
- "Spacer" (falls vorhanden) ausgewählt ist aus der Gruppe bestehend aus linearen oder verzweigten C₂-C₂₀-Alkylketten, Polyethylenglycol, Polypropylenglycol, pHPMA, PLGA, Polymer von Alkyldiamin und Kombinationen davon, wobei die Polymere 2 bis 20 Monomere aufweisen und/oder
- "M" einen zelltypspezifischen Liganden umfasst oder daraus besteht, der von einem Protein abgeleitet ist, das ausgewählt ist aus Transferrin, epidermalem Wachstumsfaktor (EGF) und basischem Fibroblastenwachstumsfaktor βFGF, einem Mono- oder Polysaccharid, das eine oder mehrere Galactose, Mannose, N-Acetylgalactosamin-Reste, Brücken-GalNac oder Mannose-6-phosphat, MTP, umfasst, ausgewählt aus SEQ ID NO: 1 bis SEQ ID NO: 7, und Vitamine wie Folsäure.

7. AAV nach einem der Ansprüche 2 bis 6, wobei:
- M ein zelltypspezifischer Ligand zum spezifischen Targeting von Hepatozyten ist und mindestens eine Einheit der Formel (III) umfasst: 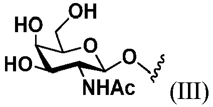 und/oder
- Spacer eine Polyethylenglycolkette ist, die 2 bis 10 Monomere umfasst.

8. AAV nach Anspruch 7, wobei das mindestens eine chemisch modifizierte Tyrosin die Formel (II) hat:

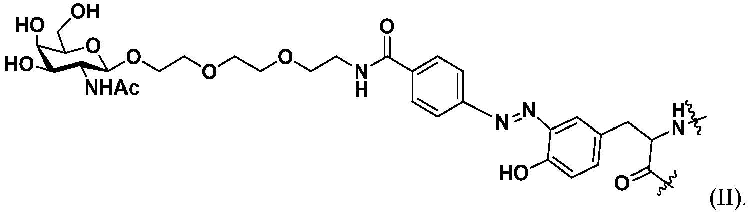

9. AAV nach einem der Ansprüche 1 bis 8, das ferner mindestens einen zusätzlichen chemisch modifizierten Aminosäurerest im Kapsid aufweist, der sich von einem Tyrosinrest unterscheidet, wobei dieser Aminosäurerest vorzugsweise eine Aminogruppe trägt, die chemisch mit einer Gruppe der Formel (V) modifiziert ist: 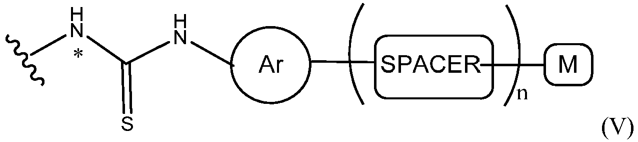 wobei:
- N* der Stickstoff der Aminogruppe eines Aminosäurerests ist, z. B. eines Lysinrests oder Argininrests,
- Ar, Spacer, n und M die gleiche Definition haben wie Ar, Spacer, n und M der Formel (II) von Anspruch 2.

10. AAV nach einem der Ansprüche 1-9, wobei das AAV ein rekombinantes AAV ist, vorzugsweise ausgewählt aus AAV mit einem Wildtyp-Kapsid, natürlich vorkommendem Serotyp-AAV, Varianten-AAV, Pseudotyp-AAV, AAV mit hybriden oder mutierten Kapsiden und selbst- komplementärem AAV.

11. Verfahren zum chemischen Modifizieren des Kapsids eines AAV, genauer gesagt zum chemischen Modifizieren mindestens eines Tyrosinrests im Kapsid eines AAV, umfassend das Inkubieren dieses AAV mit einem chemischen Reagens, das eine reaktive Gruppe trägt, ausgewählt aus einem Aryldiazonium, und einer 4-Phenyl-1,2,4-triazol-3,5-dion (PTAD)-Einheit unter Bedingungen, die für die Umsetzung dieser reaktiven Gruppe mit einem im Kapsid des AAV vorhandenen Tyrosinrest förderlich sind, um eine kovalente Bindung zu bilden.

12. Verfahren nach Anspruch 11, das das Inkubieren des AAV mit einem chemischen Reagenz der Formel (VId) umfasst. 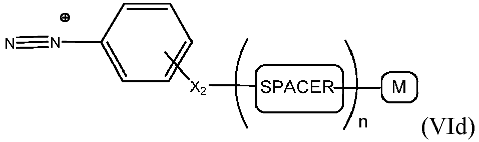 um mindestens einen chemisch modifizierten Tyrosinrest im Kapsid der Formel (Ic) zu erhalten: 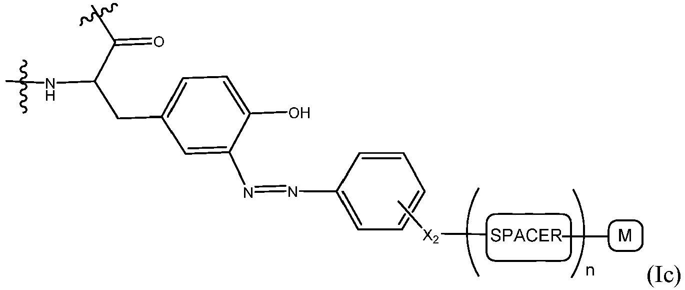 wobei:
- X₂ -C(=O)-NH, -C(=O)-O, -C(=O)-O-C(=O)-, O-(C=O)-, NH-C(=O)-, NH-C(=O)-NH, -O-C=O-O-, O, NH, -C=S-NH oder NH-C=S- ist, vorzugsweise -(C=O)-NH- oder -(C=O)-O-
- X₂ in para-, meta- oder ortho-Stellung , vorzugsweise in para-Stellung der Phenylgruppe steht,
- Spacer eine Kohlenwasserstoffgruppe ist, die 2 bis 400 Kohlenstoffatome umfasst,
- n 0 oder 1 ist, und
- M eine chemische Einheit ist, die ein sterisches Abschirmungsmittel, ein Markierungsmittel, einen zelltypspezifischen Liganden oder eine Arzneimitteleinheit umfasst.

13. Pharmazeutische Zusammensetzung, umfassend ein AAV wie in einem der Ansprüche 1-10 definiert und mindestens einen pharmazeutisch verträglichen Exzipienten.

14. AAV nach den Ansprüchen 1-10 oder eine pharmazeutische Zusammensetzung nach Anspruch 13 zur Verwendung als diagnostisches Mittel oder als Arzneimittel *in vivo*, vorzugsweise in der Gentherapie

15. Verwendung eines AAV nach Anspruch 1-10 oder einer pharmazeutischen Zusammensetzung nach Anspruch 13 als diagnostisches Mittel und als Genvektor *ex vivo* oder *in vitro.*

## Revendications

1. Virus adéno-associé (AAV) ayant au moins un résidu de tyrosine chimiquement modifié dans sa capside, dans lequel ledit résidu de tyrosine chimiquement modifié est de formule (I) : 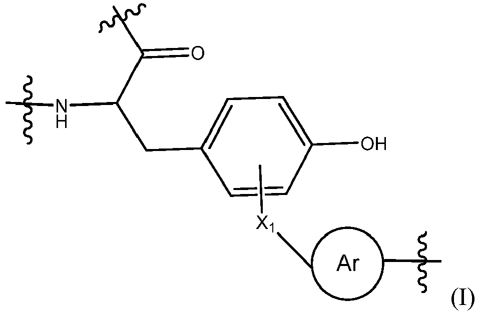 dans laquelle :
- X₁ est choisi dans le groupe constitué par : 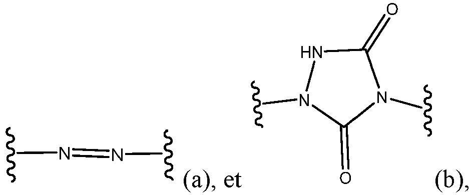 and
- Ar est un fragment aryle ou hétéroaryle éventuellement substitué.

2. AAV selon la revendication 1, dans lequel l'au moins un résidu de tyrosine chimiquement modifié est de formule (Ia) : 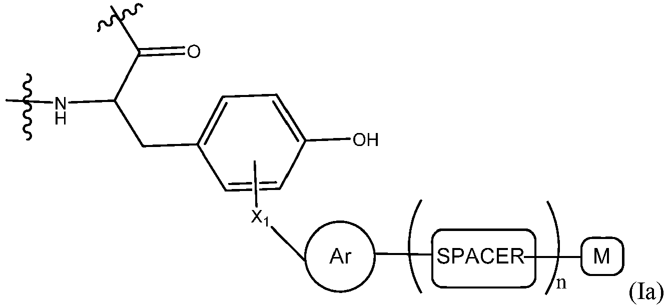 dans laquelle
- X₁ et Ar sont tels que définis dans la revendication 1,
- SPACER est un groupe pour lier le groupe "Ar" au fragment fonctionnel "M" qui de préférence comprend jusqu'à 1000 atomes de carbone et qui est de préférence sous la forme d'une chaîne chimique qui comprend éventuellement des hétéroatomes et/ou des fragments cycliques,
- n vaut 0 ou 1, et
- M est un fragment fonctionnel comprenant un agent d'encombrement stérique, un agent de marquage, un ligand à spécificité de type cellulaire ou un fragment médicament.

3. AAV selon la revendication 2, dans lequel X₁ est 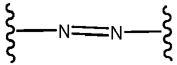 et/ou "Ar" est choisi parmi les radicaux substitués ou non substitués phényle, pyridyle, naphtyle, et anthracényle.

4. AAV selon l'une quelconque des revendications 1 à 3, dans lequel l'au moins un résidu de tyrosine chimiquement modifié est de formule (Ic) : 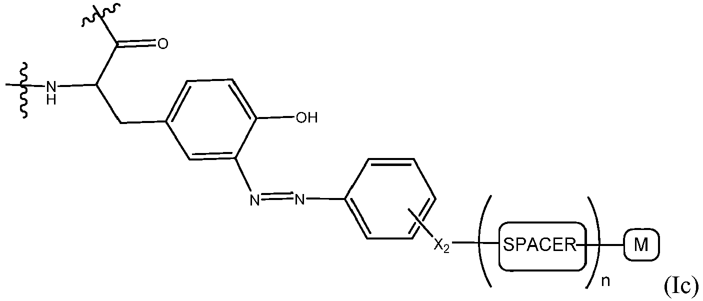 dans laquelle :
- X₂ est -C(=O)-NH, -C(=O)-O, -C(=O)-O-C(=O)-, O-(C=O)-, NH-C(=O)-, NH-C(=O)-NH, -O-C=O-O-, O, NH, -NH(C=S)- ou -(C=S)-NH-, de préférence -(C=O)-NH- ou -(C=O)-O-
- X₂ est en position para, méta ou ortho, de préférence en para du groupe phényle,
- SPACER, n et M sont tels que définis dans la revendication 2.

5. AAV selon l'une quelconque des revendications 2 à 4, dans lequel :
- " SPACER", quand il est présent, est choisi dans le groupe constitué par les chaînes hydrocarbonées en C₂ à C₄₀ linéaires ou ramifiées, saturées ou insaturées, éventuellement substituées, le polyéthylèneglycol, le polypropylèneglycol, le pHPMA, le PLGA, les polymères d'alkyldiamines et leurs combinaisons, et/ou
- "M" comprend, ou consiste en, un ligand de ciblage de type cellulaire, de préférence choisi parmi un mono- ou poly-saccharide, une hormone, y compris une hormone stéroïde, un peptide tel qu'un peptide RGD, un peptide de ciblage musculaire (MTP) ou l'Angiopep-2, une protéine ou un fragment de celle-ci, un récepteur membranaire ou un fragment de celui-ci, un aptamère, un anticorps y compris un anticorps à chaîne lourde, et des fragments de celui-ci tels que Fab, Fab', et VHH, un ScFv, un Spiegelmer, un aptamère peptidique, les vitamines, et les médicaments tels que les ligands de CB1 et/ou CB2.

6. AAV selon l'une quelconque des revendications 2 à 4, dans lequel :
- " SPACER", quand il est présent, est choisi dans le groupe constitué par les chaînes alkyle en C₂ à C₂₀ linéaires ou ramifiées, le polyéthylèneglycol, le polypropylèneglycol, le pHPMA, le PLGA, les polymères d'alkyldiamines et leurs combinaisons, lesdits polymères ayant de 2 à 20 monomères, et/ou
- "M" comprend, ou consiste en, un ligand à spécificité de type cellulaire dérivé d'une protéine choisie parmi la transferrine, le facteur de croissance épidermique (EGF), et le facteur de croissance des fibroblastes basique βFGF, un mono- ou poly-saccharide comprenant un ou plusieurs résidus de galactose, mannose, N-acétylgalactosamine, un pont GalNac, ou le mannose-6-phosphate, les MTP choisis parmi les SEQ ID NO : 1 à 7, et les vitamines telles que l'acide folique.

7. AAV selon l'une quelconque des revendications 2 à 6, dans lequel :
- M est un ligand à spécificité de type cellulaire pour cibler spécifiquement les hépatocytes, et comprend au moins un fragment de formule (III) : 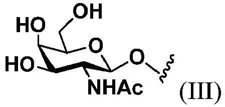 et/ou
- SPACER est une chaîne de polyéthylèneglycol comprenant de 2 à 10 monomères.

8. AAV selon la revendication 7, dans lequel ladite au moins une tyrosine chimiquement modifiée est de formule (II) :

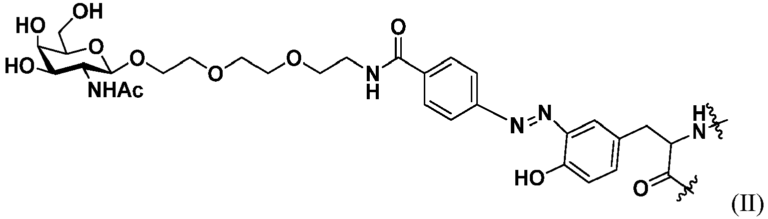

9. AAV selon l'une quelconque des revendications 1 à 8, qui a en outre au moins un résidu d'acide aminé chimiquement modifié additionnel dans la capside, qui est différent d'un résidu de tyrosine, ledit résidu d'acide aminé portant de préférence un groupe amino chimiquement modifié par un groupe de formule (V) : 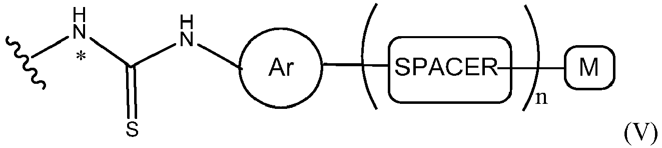 dans laquelle :
- N* est l'azote du groupe amino d'un résidu d'acide aminé, par exemple d'un résidu de lysine ou d'un résidu d'arginine,
- Ar, SPACER, n et M ont les mêmes définitions que Ar, SPACER, n et M dans la formule (II) de la revendication 2.

10. AAV selon l'une quelconque des revendications 1 à 9, lequel AAV est un AAV recombinant, de préférence choisi parmi les AAV ayant une capside de type sauvage, les AAV de sérotypes naturels, les AAV variants, les AAV pseudotypiques, les AAV avec des capsides hybrides ou mutées, et les AAV auto-complémentaires.

11. Méthode pour modifier chimiquement la capside d'un AAV, plus précisément pour modifier chimiquement au moins un résidu de tyrosine dans la capside d'un AAV, qui comprend l'incubation dudit AAV avec un réactif chimique portant un groupe réactif choisi parmi un fragment diaryl-azonium, et un fragment 4-phényl-1,2,4-triazole-3,5-dione (PTAD) dans des conditions propices à la réaction dudit groupe réactif avec un résidu de tyrosine présent dans la capside du AAV de façon à former une liaison covalente.

12. Méthode selon la revendication 11, qui comprend l'incubation du AAV avec un réactif chimique de formule (VId) 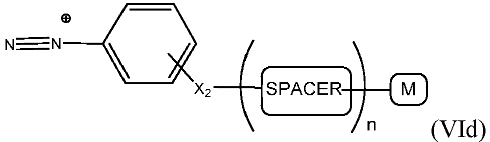 de façon que soit obtenu au moins un résidu de tyrosine chimiquement modifié dans la capside de formule (Ic) : 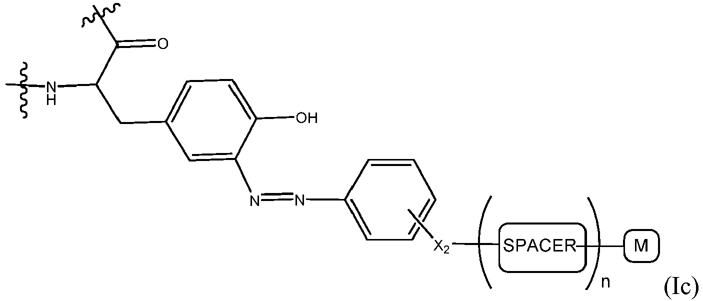 dans laquelle :
- X₂ est -C(=O)-NH, -C(=O)-O, -C(=O)-O-C(=O)-, O-(C=O)-, NH-C(=O)-, NH-C(=O)-NH, -O-C=O-O-, O, NH, -C=S-NH ou NH-C=S-, de préférence -(C=O)-NH- ou -(C=O)-O-
- X₂ est en position para, méta ou ortho, de préférence en para du groupe phényle,
- SPACER est un groupe hydrocarboné comprenant de 2 à 400 atomes de carbone,
- n vaut 0 ou 1, et
- M est un fragment fonctionnel comprenant un agent d'encombrement stérique, un agent de marquage, un ligand à spécificité de type cellulaire ou un fragment médicament.

13. Composition pharmaceutique comprenant un AAV tel que défini dans l'une quelconque des revendications 1 à 10 et au moins un excipient pharmaceutiquement acceptable.

14. AAV selon les revendications 1 à 10 ou composition pharmaceutique selon la revendication 13, pour une utilisation en tant qu'agent diagnostique ou médicament *in vivo*, de préférence en thérapie génique.

15. Utilisation d'un AAV selon les revendications 1 à 10 ou d'une composition pharmaceutique selon la revendication 13 en tant qu'agent diagnostique et en tant que vecteur génique *ex vivo* ou *in vitro.*
